# EUROPEAN PATENT SPECIFICATION

(11) **EP 0 918 545 B1**
(45) Date of publication and mention of the grant of the patent: **12.07.2000**
(21) Application number: 97945975.7
(22) Date of filing: 28.11.1997
(51) Int. Cl.: A61K 47/48

(54) **TUMOUR THERAPY**
KREBS THERAPIE
THERAPIE TUMORALE

(30) Priority: 30.11.1996 GB 9624993
(43) Date of publication of application: 02.06.1999
(73) Proprietor: ENZACTA R & D LIMITED, London WC1E 6HQ (GB)
(72) Inventor: BAGSHAWE, Kenneth, Dawson, London W1H 5TA (GB)
(74) Representative: Miles, John Stephen
(86) International application number: GB9703284
(87) International publication number: WO9824478

(56) References cited:
- WO-A-89/10140
- WO-A-93/13806
- WO-A-97/24143
- SATCHI, R. ET AL: "PDEPT: polymer directed enzyme prodrug therapy" PROC. INT. SYMP. CONTROLLED RELEASE BIOACT. MATER., 1997, 24TH,, 773-774, XP002064122
- SHERWOOD R. F.: "Advanced drug delivery reviews: enzyme prodrug therapy" ADV DRUG DELIV REVIEWS, vol. 22, 1996, pages 269-288, XP002064128
- SHARMA S. K.: "Accelerated clearance systems" ADV DRUG DELIV REVIEWS, vol. 22, 1996, pages 315-324, XP002064129
- DUNCAN, R._CONNORS, T. A._MEADA, H.: "DRUG TARGETING IN CANCER THERAPY: THE MAGIC BULLET" JOURNAL OF DRUG TARGETING, pages 317-319, XP002064126
- NUCCI M. L. ET AL: "The therapeutic value of poly(ethylenglycol) modified proteins" ADVANCED DRUG DELIVERY REVIEWS, vol. 6, no. 2, April 1991, pages 133-153, XP002064123

## Description

The targeting of cancers with drugs, radioisotopes and toxins has made use of antibodies directed at tumour associated antigens or fragments of such antibodies. In general, antibodies directed at tumour associated antigens accumulate in tumours more effectively than any other class of molecule which have no known basis for binding to tumour cells. The process of accumulation of molecules within tumour masses and factors affecting their distribution have been described by various authors (Jain, 1989, Szerkerke & Driscoll, 1977). Expressed as a percentage of administered dose per gram of tumour in nude mice bearing human cancer xenografts, values for antibodies generally fall in the range 2-10%. In the human, values are about 1000-fold less (Mach *et al,* 1980, Dykes *et al,* 1987) but, by scaling up the dose proportionate to body mass, antibody concentrations in tumour attain comparable values to those obtained in the mouse model.

Conjugates prepared by linking drugs to antibodies or antibody fragments have been claimed to be more efficacious than conventional drug administration (Garnett & Baldwin, 1986) but none have so far achieved clinical acceptance. Large molecules such as antibodies tend to deliver less drug than can be delivered by conventional free drug administration because the amount of drug that can be attached to an antibody without loss of binding activity is limited, the large size of antibodies limits the amount of antibody binding to tumours and antibody-drug conjugates often need to be internalised to release the drug.

To overcome this limitation of drug delivery the concept has been advanced of linking an enzyme to an antibody or antibody fragment (Bagshawe, 1987, Bagshawe *et al,* 1988, Senter *et al,* 1988) and, after allowing time for the enzyme to localise at tumour sites, administering a prodrug which is a substrate for the enzyme, the end product of the catalysis being a cytotoxic compound. The object of the approach is to maximise the concentration of drug within tumours and to minimise the concentration of drug in normal tissues. The system has been described as "antibody directed enzyme prodrug therapy" (ADEPT). It was clear at the outset of developing the ADEPT approach that there were three main problems to be overcome (Bagshawe, 1989). The first was the requirement for prodrugs which would generate drugs with a short half-life so as to minimise the amount of drug leaking from the tumour sites, which then affected vulnerable normal tissues such as bone marrow. Secondly, it was necessary to ensure that enzyme was not present in normal tissues when prodrug was administered since this would allow generation of cytotoxic drug in normal tissues and would be dose limiting. Thirdly, it was recognised that it was advantageous to conjugate enzymes derived from non-human species that have no human homolog so as to avoid activation by endogenous enzymes. However, the specificity of action conferred by bacterial enzymes, for instance, carried the penalty of high immunogenicity.

Although early prodrugs made for the ADEPT approach generated drugs with long half-lives, prodrugs generating drugs with short half-lives have subsequently been developed (Blakey *et al,* 1996, Jungheim & Shepherd, 1994).

The issue of ensuring that there was little or no enzyme present in blood and normal tissues when prodrug was administered was open to a range of possible solutions (Sharma *et al,* 1994). One method which proved effective was administration of a monoclonal antibody directed at the enzyme after the antibody-enzyme conjugate had localised in tumour and before the prodrug was given (Bagshawe, 1989; WO 89/10140; Sharma *et al,* 1994). An antibody which inactivated the enzyme was found to be most efficient. Such an antibody could inactivate enzyme in blood, in normal tissues and at tumour sites. To accelerate clearance of the enzyme-inactivating antibody from the blood, and thereby to minimise its penetration of the tumour, additional galactose residues were attached to the antibody. When the galactosylated anti-enzyme antibody was given, it resulted in rapid clearance from the blood of the galactosylated antibody and of complexes formed between it and the antibody-enzyme conjugate. The evidence is consistent with the anti-enzyme antibody and complex being taken up by hepatocytes which express galactose receptors. Tumours removed from nude mice which had received antibody-enzyme conjugate followed by galactosylated anti-enzyme antibody showed no significant reduction in enzyme activity although enzyme activity in blood was reduced quickly to values below the sensitivity limit of the spectrophotometric method in use. When used clinically, the results on blood clearance of enzyme activity were similar to those obtained in the mouse.

There have been several variations of the basic ADEPT approach. In WO 93/13805 (Bagshawe), the enzyme, instead of converting a less cytotoxic prodrug into a more cytotoxic drug, locally converted a systemically-provided inhibitor of a cytotoxic compound into a less inhibitory compound. In WO 93/13806 (Bagshawe), an inhibitor of a cytotoxic compound (generated locally by the ADEPT approach) was restrained in the vascular compartment, so that the cytotoxic compound was inhibited or inactivated when it left the tumour site.

In all of these ADEPT-type publications, the delivery of the enzyme to the tumour was achieved by using a tumour-specific antibody or fragment or variant thereof.

Although antibodies and their fragments directed at tumour associated antigens accumulate at tumour sites expressing the target antigen in greater concentration than any other class of molecule when measured in comparison with normal tissue concentrations of the same antibody or fragment, it has long been known (Duran-Reynolds, 1939) that macromolecules accumulate non-specifically in tumours. This non-specific accumulation of macromolecules is actually one component in the favourable tumour:non-tumour distribution of antibodies directed at tumour associated antigens. Macromolecules known to accumulate in tumours non-specifically include albumin, immunoglobulins, transferrin, liposomes, nanoparticles and biodegradable polymers including dextrans, polyethylene glycol, polylysine and hydroxypropylmethylacrylamide. Macromolecules accumulate in human xenografted tumours in nude mice up to about 2.0% of administered dose per gram of tumour. Macromolecules such as polyethylene glycol and dextrans have been found to modify the clearance rate of substances to which they are attached and modify their concentration in tumours (Melton *et al,* 1987, Eno-Amooquaye *et al,* 1996). In exceptional tumours a non-specific macromolecule may accumulate in greater concentration than an antibody directed at the secreted antigen (Searle *et al,* 1981).

The discovery that such macromolecules accumulate in solid tumour tissue has been called the "enhanced permeability and retention" effect (EPR effect) and is attributed to the 'leakiness' of tumour capillaries and deficient lymphatic drainage (Matsumura, Y. & Macda H., 1986).

Macromolecules have been employed as carriers for drugs (Blair & Ghose, 1983) and claims have been made for the superior efficacy of macromolecule-linked drugs over free drugs (Duncan *et al,* 1988). In part the superior efficacy is based on the reduced toxicity of the conjugated drug. The differential accumulation of macromolecules between tumour and non-tumour tissues may not ensure that the concentration of drug delivered by macromolecules exceeds that delivered by free drug administration. This results from the limited number of drug molecules that can be attached to a macromolecule and the smaller number of macromolecules that can be delivered to any tissue compared with a low molecular weight drug; that is, the molar concentration of macromolecules is lower. This may be why those working in this field have not envisaged using non-specific uptake of macromolecules to deliver the enzyme in an ADEPT-type approach.

US 5,561,119 to Jacquesy *et al* relates to glycosylated prodrugs and their use with tumour-specific immunoenzymatic conjugates for the treatment of cancer.

Seymour (1992) *Crit. Rev. Ther. Drug Carrier Systems 9,* 135-187 discusses the accumulation of soluble macromolecules and drug conjugates in tumours.

EP 0 028 917 discusses the use of lipid vesicles to deliver a tracer molecule or drug. The vesicles accumulate preferentially in the lymphatic system and/or the lung, spleen or liver.

EP 0 142 905 discusses the limitations of immune targeting to tumour cells, particularly those caused by the heterogeneity of tumour cells, and suggests a method using immune targeting to deliver a cytotoxic agent to a tumour region, wherein the toxic effect is not limited to the cells bearing the appropriate antigen.

WO 91/08770 discusses the accumulation of a diagnostic or therapeutic agent at a tumour site where the accumulation is a result of cleavage of a soluble substrate-agent complex by an antibody enzyme conjugate immobilised at a site to which an antibody can selectively bind.

CA 1 227 427 describes the synergistic effect on tumour progression of administering chondroitin poly sulphate, trypsin and cyclosphosphamide; the three agents are not linked and accumulation in a tumour is not described.

Pimm & Hudecz (1996) *J. Cancer Res. Clin. Oncol.* **122**, 45-54 describes the distribution of radiolabelled macromolecules in mice.

WO 92/13570 relates to complexes that facilitate uptake of nucleic acids by eukaryotic cells.

Macromolecules in the form of polymers have been attached to enzymes including L-asparaginase (Keating *et al* (1993) *Leukaemia Lymphoma* **10**, 153-157), uricase, (Chua *et al* (1988) *Annals Int. Med.* **109(2)**, 114-117), phenylalanmine ammonia lyase, (Wieder *et al* (1979) *J. Biol. Chem.* **254**, 12579-12587), glutaminase-asparaginase (Abuchowski *et al* (1981) *Cancer Treat. Rep.* **65,** 1077-1081), and tryptophanase (Yoshimoto *et al* (1985) *Enzyme* **36**, 261). In all these examples the purpose was for the enzyme to exert an effect on naturally occurring substrates in tissues.

### Summary of the Invention

I have now found that non-specific (EPR effect) uptake of a macromolecule by a tumour is enough to deliver an appropriate level of the enzyme in an ADEPT-type of system and that an adequate ratio of tumour-associated cytotoxic drug to non-tumour-associated drug can be achieved if the enzyme-macromolecule conjugate is cleared or inhibited when away from the tumour. This approach is applicable to any of the ADEPT systems described above but should perhaps be called MDEPT (macromolecule directed enzyme prodrug therapy).

Accordingly, one aspect of the invention provides a three component therapeutic system comprising (i) a first compound, (ii) a catalytic macromolecule which does not bind specifically to a tumour antigen but which is capable, following administration to the vascular compartment of a mammal, of being taken up by a tumour in the mammal and is capable of converting the first compound into a second compound, and (iii) an inhibitor which, following administration to the said vascular compartment, reduces the level of the said catalytic conversion activity in the vascular compartment and normal tissues.

The term "tumour" is to be understood as referring to all forms of neoplastic cell growth, including tumours of the lung, liver, blood cells, skin, pancreas, stomach, colon, prostate, uterus, breast, lymph glands and bladder. Solid tumours are especially suitable.

The potential advantages in using non-antibody macromolecules for this purpose are substantial. Firstly, a non-specific macromolecule may be selected that is non-immunogenic. Secondly, a macromolecule may be much less costly to produce than humanised antitumour antibody. Thirdly, it has been shown that some polymers reduce or eliminate the immunogenicity of proteins, including enzymes, attached to them (Abuchowski *et al,* 1977, Wileman *et al,* 1986, Mikolajczyk *et al,* 1996). Fourthly, whereas an antibody binds to only a limited range of cancers (antibodies only exist for about 60% of all malignancies), the macromolecule uptake by tumours appears to be a characteristic common to all solid cancers so far examined.

Thus, many tumours such as sarcomas for which no selective antibodies have yet been reported may be targeted in this principle.

The relatively low differential between tumour and non-tumour tissues with non-specific macromolecules is exploitable only if the level of normal tissue enzyme is inhibited, for example by using a galactosylated anti-enzyme antibody. To get the required amount of enzyme to tumour sites when the enzyme is conjugated to a non-specific macromolecule will require a greater amount of such a conjugate to be administered than would be the case with a specific antibody-enzyme conjugate, but the lower cost of the former should offset its lower efficiency.

Preferably, the macromolecule used in the invention is hydrophilic and is characterised by being soluble in body fluids and in conventional fluids for parenteral administration. Suitably, the macromolecule is biodegradable so that systemic accumulation during repeated administration is avoided. Clearly, however, it must not be degraded so fast as to fail to accumulate at the tumour site. Preferably, when conjugated to the selected enzyme, the molecular weight and size of the conjugate exceeds that of the renal threshold for urinary excretion (MW 60 000), as this helps the blood concentration to be sufficient to provide an effective blood:tumour concentration gradient. A molecular weight of up to at least 800 000 is generally suitable, for example up to 160 000. The macromolecule is preferably one which is not readily captured by the reticuloendothelial system. To make it catalytic, the macromolecule may be conjugated to one or more enzyme molecules by simple chemical methods, using bi-functional agents which do not degrade the attached enzyme. Preferably, the starting macromolecule confers reduced immunogenicity on an immunogenic enzyme to which it is conjugated.

The molecular weights given exclude any water of hydration.

Macromolecules that are available as sub-units and are not biodegradable may be linked by biodegradable linking units so that the non-biodegradable components are filtered through the kidneys and excreted in the urine.

Alternatively, it is preferred if the polymer used to make the macromolecule is not biodegradable then the molecular weight of any non-biodegradable portion of the conjugate should be less than the renal threshold (circa 70000) so that after degradation of the biodegradable portion the residual non-biodegradeable portion is excreted through the kidneys.

Whereas some macromolecules are not known to be internalised by cells others, such as N-(2-hydroxypropyl)methylacrylamide, are internalised through more than one mechanism (Duncan *et al,* 1996).

Preferably, the macromolecule is polyethylene glycol (PEG). Derivatisation of proteins with polyethylene glycol has been demonstrated numerous times to increase their blood circulation lifetimes as well as decrease their antigenicity and immunogenicity. A wide variety of methods has been developed to couple PEG to proteins and these are summarised in Table 1.

Conveniently, the macromolecule may, to the exclusion of transferrin, be any of a dextran; a polyamino acid; or a non-tumour-specific protein such as an immunoglobulin or an albumin. Suitably, it may be a copolymer of styrene and maleic anhydride, or may be polyaspartic acid, poly-L-lysine or polyethyleneimine.

The catalytic macromolecule is not specific for the tumour. Hence, for example, it does not consist of an antibody or part thereof which binds specifically to a tumour-associated antigen. It may nevertheless comprise non-specific immunoglobulins.

The advantage of being able to use a non-antibody macromolecule for targeting an enzyme to tumour sites extends to other applications of tumour located enzymes. Some molecules, in particular some polymers, are known to be readily internalised by tumour cells so that advantage can be taken of enzymes, such as nitroreductase, that require intracellular co-factors, such as NAD(P)H (the reduced form of nicotinamide adenine dinucleotide (phosphate)), to activate prodrugs that are also internalised and convert them to highly potent cytotoxic substances. Such cytotoxic agents are therefore able to kill the cells which internalise the macromolecule enzyme and the prodrug and to release sufficient toxic drug into the extracellular space to achieve a concentration of cytotoxic drug that is lethal to adjacent cells (see US Patent Application No 60/009,361, filed December 29, 1995, incorporated herein by reference which is published as WO 97/24143; PCT/GB96/03254). For example, a conjugate may be formed between *E. coli* nitroreductase and the macromolecule (for example hydroxypropylmethylacrylamide), which is internalised in cells.

Optionally after inactivation or removal of intravascular nitroreductase (for example with anti-nitroreductase antibody, preferably polygalactosylated) (see further below), the pro-drug CB 1954 (Knox *et al* (1993) *Cancer & Metastasis Rev.* **12**, 195-212) is administered, and is selectively activated in the tumour cells because (i) the tumour cells have a higher level of the nitroreductase, and (ii) the nitroreductase functions only in the presence of the (intracellular) co-factor NAD(P)H or NRH (nicotinamide riboside). The benzyloxycarbonyl derivative of actinomycin D can also be used as a prodrug (releasing actinomycin D, see Mauger *et al* (1994) J. *Med. Chem.* **37**, 3452-3458) with this reductase-based system.

In a preferred embodiment of the invention, a human enzyme NQO2 is used. NQO2 is NAD(P)H:quinone oxidoreductase 2, and its use in tumour targeted therapy in conjunction with a prodrug such as CB 1954 (5-(aziridin-1-yl)-2,4-dinitrobenzamide) is described in the patent application GB 9712370.7, incorporated herein by reference. Preferably, in this embodiment, a co-factor is used which is excluded from cells, such as nicotinic acid.

The cDNA encoding human NAD(P)H:quinone reductase 2 (NQO2) is given in Jaiswal *et al* (1990) *Biochemistry* **29,** 1899-1906 and the gene structure of the NQO2 gene is given in Jaiswal (1994) J. *Biol. Chem.* **269**, 14502-14508, both of which are incorporated herein by reference. The skilled person can readily obtain and manipulate DNA encoding NQO2 based on the teachings contained herein using genetic engineering and recombinant DNA techniques which are well known, some of which are described in Sambrook *et al* (1989), "Molecular cloning, a laboratory manual", Cold Spring Harbor Laboratory Press, Cold Spring Harbor, New York.

A variant or fragment or fusion or derivative of NQO2 may be used in place of NQO2 with the given amino acid sequence provided that it has substantially the same activity as NQO2 towards a given prodrug. The enzyme NQO2 catalyses the conversion of, for example, the prodrug CB 1954 and prodrug analogues thereof. Thus, preferably the variant or fragment or fusion or derivative of NQO2 has substantially the same activity towards CB 1954 as does NQO2 itself. Conveniently, the said variant or fragment or fusion or derivative has at least 0.1 x the k_{cat}/Kₘ of NQO2, more preferably at least 0.5 x and still more preferably at least 0.9 x the k_{cat}/Kₘ of NQO2.

Preferably, the variant or fragment or fusion or derivative of NQO2 also has substantially the same cofactor specificity. Preferably the variant or fragment or fusion or derivative of NQO2 can use nicotinic acid as a cofactor. Preferably, the variant or fragment or fusion or derivative of NQO2 binds the cofactor at least 0.1 x as well as NQO2 itself, more preferably at least 0.5 x as well and still more preferably at least 0.9 x as well.

Preferably, activation of the prodrug by NQO2 occurs in the extracellular space and a cofactor, such as nicotinic acid, is used which is not internalised into the cell.

When the present invention is applied to the basic ADEPT approach or any of the developments thereof mentioned above, the enzyme and pro-drug system may be any of those previously proposed. The cytotoxic substance may be any existing anti-cancer drug such as an alkylating agent; an agent which intercalates in DNA; an agent which inhibits any key enzymes such as dihydrofolate reductase, thymidine synthetase, ribonucleotide reductase, nucleoside kinases or topoisomerase; or an agent which effects cell death by interacting with any other cellular constituent. Etoposide is an example of a topoisomerase inhibitor.

Reported prodrug systems include: a phenol mustard prodrug activated by an E. *coli* β-glucuronidase (Wang *et al,* 1992 and Roffler *et al,* 1991); a doxorubicin prodrug activated by a human β-glucuronidase (Bosslet *et al,* 1994); further doxorubicin prodrugs activated by coffee bean α-galactosidase (Azoulay *et al,* 1995); daunorubicin prodrugs, activated by coffee bean α-D-galactosidase (Gesson *et al,* 1994); a 5-fluorouridine prodrug activated by an E. *coli* β-D-galactosidase (Abraham *et al,* 1994); and methotrexate prodrugs (eg methotrexate-alanine) activated by carboxypeptidase A (Kuefner *et al,* 1990, Vitols *et al,* 1992 and Vitols *et al,* 1995). These and others are included in the following table.

| **Enzyme** | **Prodrug** |
|---|---|
| Carboxypeptidase G2 | Derivatives of L-glutamic acid and benzoic acid mustards, aniline mustards, phenol mustards and phenylenediamine mustards; fluorinated derivatives of these |
| | |
| Alkaline phosphatase | Etoposide phosphate |
| | |
| | Mitomycin phosphate |
| Beta-glucuronidase | *p*-Hydroxyaniline mustard-glucuronide |
| | |
| | Epirubicin-glucuronide |
| Penicillin-V-amidase | Adriamycin-N phenoxyacetyl |
| Penicillin-G-amidase | N-(4'-hydroxyphenyl acetyl) palytoxin |
| | |
| | Doxorubicin and melphalan |
| Beta-lactamase | Nitrogen mustard-cephalosporin p-phenylenediamine; doxorubicin derivatives; vinblastine derivative-cephalosporin, cephalosporin mustard; a taxol derivative |
| | |
| Beta-glucosidase | Cyanophenylmethyl-beta-D-glucopyranosiduronic acid |
| Nitroreductase | 5-(Azaridin-1-yl-)-2,4-dinitrobenzamide |
| Cytosine deaminase | 5-Fluorocytosine |
| Carboxypeptidase A | Methotrexate-alanine |

(This table is adapted from Bagshawe (1995) *Drug Dev. Res.* **34**, 220-230, from which full references for these various systems may be obtained; the taxol derivative is described in Rodrigues *et al* (1995).)

Suitable enzymes for forming part of the catalytic macromolecules of the invention include: exopeptidases, such as carboxypeptidases G, G1 and G2 (for glutamylated mustard prodrugs), carboxypeptidases A and B (for MTX-based prodrugs) and aminopeptidases (for 2-α-aminocyl MTC prodrugs); endopeptidases, such as eg thrombolysin (for thrombin prodrugs); hydrolases, such as phosphatases (eg alkaline phosphatase) or sulphatases (eg aryl sulphatases) (for phosphylated or sulphated prodrugs); amidases, such as penicillin amidases and arylacyl amidase; lactamases, such as β-lactamases; glycosidases, such as β-glucuronidase (for β-glucuronomide anthracyclines), α-galactosidase (for amygdalin) and β-galactosidase (for β-galactose anthracycline); deaminases, such as cytosine deaminase (for 5FC); kinases, such as urokinase and thymidine kinase (for gancyclovir); reductases, such as nitroreductase (for CB1954 and analogues), azoreductase (for azobenzene mustards) and DT-diaphorase and N20 N202 (both for CB1954); oxidases, such as glucose oxidase (for glucose), xanthine oxidase (for xanthine) and lactoperoxidase; DL-racemases, catalytic antibodies and cyclodextrins.

It is likely that the catalytic portion of the macromolecule, typically an enzyme or part thereof, will be active in isolation from the rest of the macromolecule but it is necessary only for it to be active when (a) it is in combination with the rest of the macromolecule and (b) the catalytic macromolecule is attached to, adjacent to or internalised in target cells.

The two functions of the catalytic macromolecule may be located in separate portions thereof. When each such portion is a polypeptide, the two portions may be linked together by any of the conventional ways of cross-linking polypeptides, such as those generally described in O'Sullivan *et al* (1979) *Anal. Biochem.* **100,** 100-108. For example, the polypeptide portion may be enriched with thiol groups and the enzyme portion reacted with a bifunctional agent capable of reacting with those thiol groups, for example the N-hydroxysuccinimide ester of iodoacetic acid (NHIA) or N-succinimidyl-3-(2-pyridyldithio)propionate (SPDP). Amide and thioether bonds, for example achieved with m-maleimidobenzoyl-N-hydroxysuccinimide ester, are generally more stable *in vivo* than disulphide bonds.

Alternatively, the compound may be produced as a fusion compound by recombinant DNA techniques whereby a length of DNA comprises respective regions encoding the two portions of the compound of the invention either adjacent one another or separated by a region encoding a linker peptide which does not destroy the desired properties of the compound. Conceivably, the two portions of the compound may overlap wholly or partly.

The DNA is then expressed in a suitable host to produce a polypeptide comprising the compound of the invention.

An important part of the three component system of the invention is the inhibitor. This inhibits the activity of, or reduces the amount of, the enzyme in the vascular compartment, which results in reduction of enzyme in normal tissues thereby providing for an appropriate differential between the amount of or effect of enzyme in the tumour and in the rest of the body. Inactivation of the catalytic site and/or clearance of the catalytic macromolecule may be achieved as in WO 89/10140.

For example, an antibody may be used, which binds at or near the catalytic site and thereby blocks it; such antibodies (preferably monoclonal or humanized) may be raised or expressed by what are by now conventional techniques. Accelerated clearance of the catalytic macromolecule can be achieved by using an antibody which has additional galactose residues or other sugars added to accelerate clearance or may be desialylated. Galactosylation of the antibody results in its rapid clearance from the blood through take-up by galactose receptors on hepatocytes. Alternatively, or additionally, the antibody-enzyme conjugate is galactosylated, and given after the hepatic galactose receptors have been blocked by asialo-bovine submaxillary gland mucoprotein or antibody directed at hepatic galactose receptor or other molecule with high affinity for galactose receptor. The blocking substance is maintained in plasma for a period of up to 24 hours so that the antibody-enzyme complex localises at tumour sites but following cessation of galactose receptor blockade, the galactosylated antibody-enzyme is quickly cleared via the available galactose receptors. The antibody can be directed to any part of the catalytic macromolecule, since its function is to clear it, not (or at least not only) to block the catalytic action. Alternatively, small molecule enzyme inhibitors may be used; for example, these are described in WO 97/20580, incorporated herein by reference. For any given enzyme, the preferred small molecule inhibitors are those indicated as being preferred for that enzyme in WO 97/20580.

Preferably, the enzyme-inactivating small compound is a compound which inhibits the conversion of the first compound into the second compound to a useful extent. The extent of inhibition is preferably >5%, more preferably >10%, still more preferably >50% and most preferably >90%.

Preferably, when the catalytic macromolecule comprises an enzyme or other macromolecule with catalytic activity the said inhibitor binds to the active site of the enzyme or other macromolecule.

By "active site" we include any site on the enzyme or other macromolecule which influences the catalytic activity whether or not the site is the site of catalysis.

In further preference, the said inhibitor binds to the active site of the enzyme or other macromolecule and in still further preference the said molecule is not exposed on the surface of the enzyme or other macromolecule.

Preferably, the inhibitor is a relatively small molecule and it is further preferred if the molecule has a relative molecular mass of less than 10000, more preferably less than 5000 and most preferably less than 1000.

It is particularly preferred if the inhibitor is a substantially irreversible inhibitor. By "substantially irreversible inhibitor" we include an inhibitor which, once bound to an enzyme or other macromolecule with catalytic activity, substantially inhibits the catalytic activity and is unlikely to become unbound.

It is particularly preferred if the k_{cat} of said enzyme, or other macromolecule with catalytic activity, with respect to the inhibitor is < 10s⁻¹ , preferably < 1s⁻¹, more preferably < 0.1s⁻¹, still more preferably < 0.01s⁻¹ and most preferably substantially 0s⁻¹.

It is also particularly preferred if the Kᵢ of said enzyme, or other macromolecule with catalytic activity, with respect to the inhibitor is <100µM, preferably <1µM, more preferably <1nM and still more preferably substantially zero.

WO 97/20580 describes inhibitors of CPG2 all of which are incorporated herein by reference.

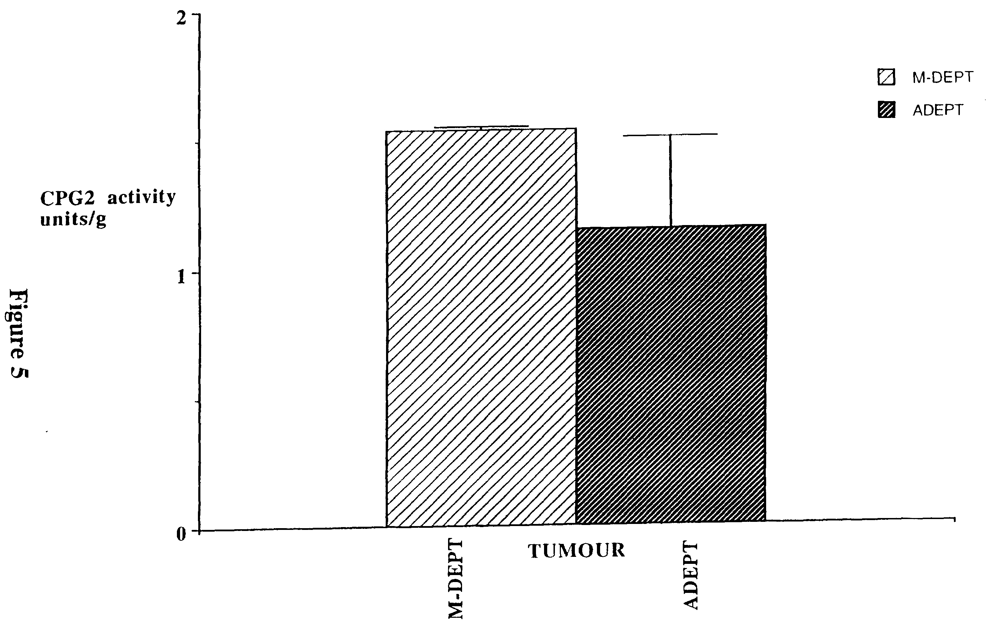

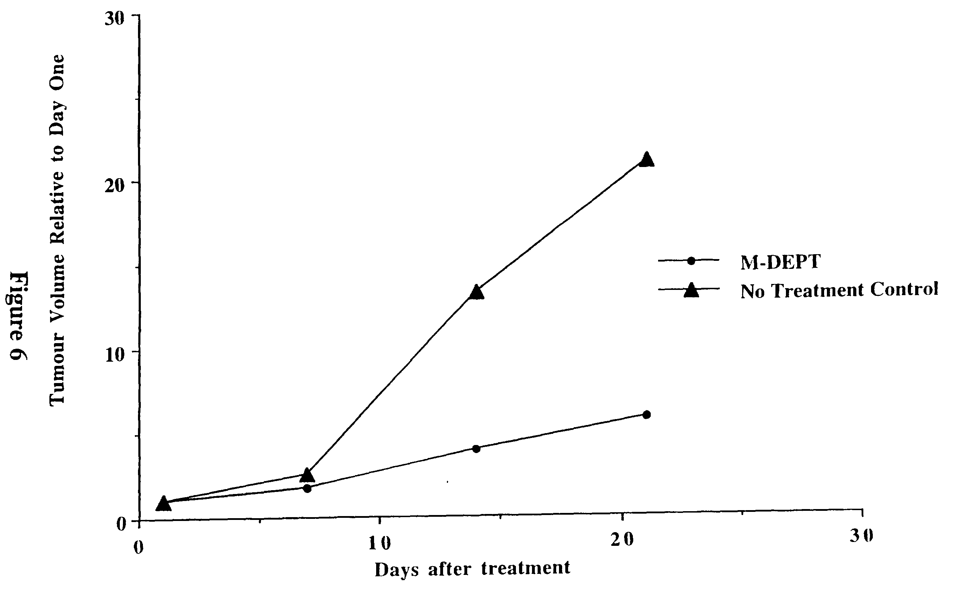

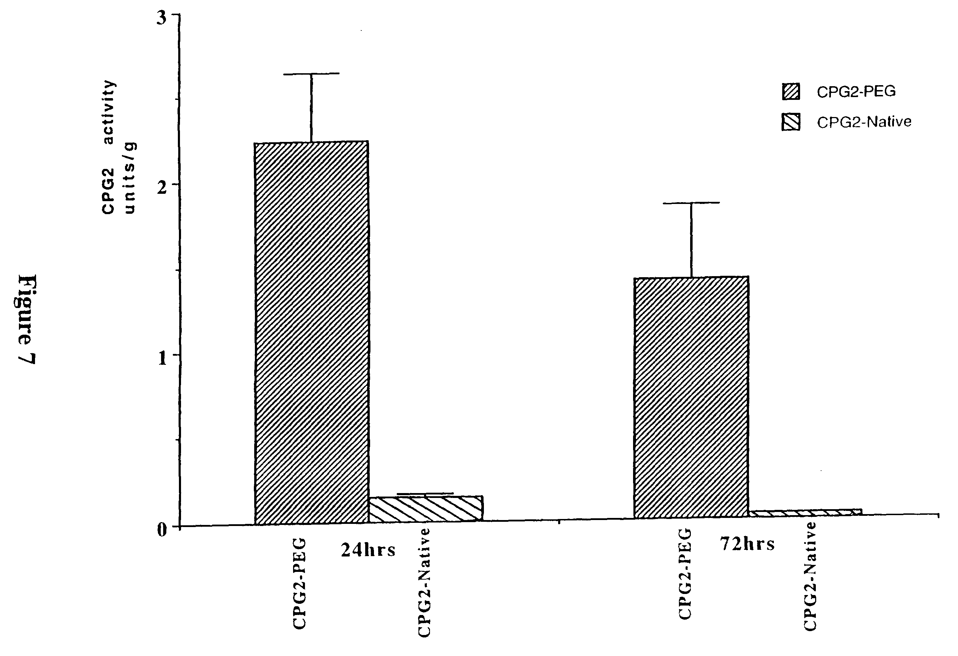

Inhibitors for use with other enzymes (as listed) include:
a) **Carboxypeptidase A** (Haenseler *et al* (1992) *Biochemistry* **31**, 214-220: Hydrolyses terminal peptide linkage adjacent to free carboxyl group. Wide specificity, maximally active with aromatic side group (Figure 6. Possible inhibitors for this enzyme are also given, Figure 7.)
b) **Glucuronidase** (Mitaku *et al* (1994) *Ann. Oncol.* **5** (Suppl. 5), 76: Sugar lactones are known to be inhibitors of this enzyme, such as D-saccharic acid-1,4-lactone for β-glucuronidase.
c) **β-Lactamase** (Svensson *et al* (1993) *Bioconj. Chem.* **3**, 176-181: Clavulanic acid is a known inhibitor of this enzyme. Other structures are also known sulbactam, thienamycin and imipenem. The potent antibiotics in this family possess β-lactamase inhibitory properties and thus run into thousands of derivatives. However, the above compounds are the most potent known at present.

It will be appreciated that salts of the inhibitor molecules may be used in the practice of the invention.

In a further embodiment it is preferred that the inhibitor is provided in the form of a precursor.

Suitably, the precursor of said inhibitor comprises said molecule in a form capable of releasing said inhibitor in a host, such as a patient. Thus, the precursor may comprise said inhibitor bound to an entity through a linkage, said linkage being biodegradable (and therefore cleavable within the host) or the precursor may comprise said inhibitor bound to an entity, whether or not through a linkage, said entity being biodegradable. In any case the inhibitor is released from the precursor in the host, such as in the patient to be treated.

The small molecule enzyme inactivating system can be further modified to encompass a biodegradable macromolecule. This embodiment has covalently attached molecules of inhibitor so that the macromolecule-inhibitor may not be able to inhibit the enzyme while it is attached to the macromolecule. However, on degradation at normal tissues the inhibitor is released and diffuses so as to inhibit any enzyme in the vicinity. The characteristics of the macromolecule-inhibitor can be chemically modified to match the location of distribution required for the therapy, thus avoiding inhibition at the tumour site. Examples of this type of system are disclosed in WO 97/20580, incorporated herein by reference.

Alternatively, a chemical moiety which reacts spontaneously with the active cytotoxic drug, or does so more effectively in the presence of an appropriate catalyst, may be used. For example, in the case of an alkylating agent as the drug, the inhibitor may comprise a thiol-containing substance and a glutathione transferase carried in or on an entity to restrain the glutathione transferase in the vascular compartment (described in more detail below). Such an inhibitor hence comprises (i) an inactivating portion (ie the chemical moiety or the enzyme which acts on the chemical moiety) and (ii) a restraining portion.

The inactivating portion may be conjugated to the restraining portion according to methods of linkage known in the art, thereby to retain the compound of the invention in the vascular compartment.

The restraining portion must be biologically compatible with all the functions of blood and the body's organs and should be biodegradable although degradation may take place slowly. It is a requirement that entry of the restraining portion into the tumour extracellular space should be minimal. It follows from the latter requirement that the inactivating portion should not be released in a free state from the restraining portion unless the free inactivating portion is rapidly removed from the blood thus limiting its access to tumour extracellular space.

The restraining portion may be a red blood corpuscle. Suitably, the compound may be contained on or within red blood corpuscles by utilising techniques known in the art for causing molecules to enter into red blood corpuscles. The inactivating portion may be attached to the erythrocyte membrane or may be made to enter the erythrocyte and be retained therein.

A method of introducing desired agents into mammalian red blood cells without unacceptable loss of cell contents has been described (US 4931276 incorporated herein by reference). This method comprises (a) suspending and incubating the cells in a solution containing a compound which readily diffuses into and out of the cells, the concentration of the compound being sufficient to cause diffusion into the cells so that the contents of the cells become hypertonic; (b) rapidly creating a transmembrane osmotic gradient by diluting the solution containing the hypertonic cells with an isotonic aqueous medium in the presence of at least one agent to be introduced to cause diffusion of water into the cells with consequent swelling and increase in permeability of the outer membranes; and (c) maintaining the increase in permeability of the membranes for a time sufficient only to permit transport of the agent into the cells and diffusion of said compound out of the cells. This method is especially useful with the "osmotic pulse" mechanism of US4478824 for loading the cells.

US4652449 (incorporated herein by reference) describes a further method for encapsulation of a biologically-active substance in mammalian erythrocytes which comprises (a) continuously feeding an aqueous suspension of erythrocytes into the main compartment of a dialysis unit, the second compartment of which contains an aqueous solution which is hypotonic with respect to the erythrocyte suspension so as to cause lysis of the erythrocytes and (b) causing the erythrocyte lysate to be in contact or contacted with the biologically active compound(s) and then resealing the erythrocyte membranes by increasing the oncotic and/or osmotic pressure of the lysate.

Alternatively, the restraining portion may be a liposome, or a high molecular weight (> 25,000 Daltons, preferably at least 40,000 Daltons) polymer such as dextran, or a protein such as macroglobulin.

Liposomes with prolonged circulation times are also potential carriers for the inactivating portion. Liposomes with prolonged circulation times have been constructed in various ways. These include the use of ganglioside GM1, or a mixture of sphingomyelin, egg phosphatidylcholine and cholesterol, or other relatively rigid carrier lipids. For each type of liposome, optimisation by sizing is also desirable to optimise prolonged intravascular residence. Gabizon and Papahadjopoulos (1988) *Proc. Natl. Acad. Sci. USA* **85**, 6949-6953) found a mean particle diameter of 100 nm to be optimal. However, liposomes with a prolonged circulation time are suitable only if they show no significant uptake in tumours.

Dextrans are polysaccharides consisting of α-D-glucose units joined predominantly by 1-6 linkages. Partially hydrolysed and fractionated dextran has been widely used as a blood plasma expander. They are cleared by dextranases which are present in the liver, spleen, kidney, intestinal mucosa and colon (Rosenfeld *et al* (1959) Biokhimia [Engl] **24**, 965-970; Ammon (1963) *Enzymologia* **25**, 245-251; Serry & Hehre (1956) *J. Bacteriol.* **71**, 373-380). Dextrans that are retained in the vascular compartment are widely available with mean molecular weights of 40 kD (Gentran 40, Rheomacrodex), 70-75 kD (Gentran 70 Macrodex) and 110 kD. They are potentially antigenic and some people have pre-existing antibodies, but reactions to dextrans are reported to be no higher than to other widely used pharmaceutical agents and are mild in character (Goodman and Gilman. *The Pharmacological Basis of Therapeutics.* 8th Edition, Pergamon Press, New York, Oxford, 1990).

Other polymeric drug carriers have been developed from carbohydrates, peptides and lipids. Some of these may be suitable alternative restraining portions for the inactivating portion provided they fulfill the criteria defined here.

The restraining portion may advantageously have a negative charge and may have low lipophilicity. Suitably, the restraining portion is biodegradable. By "biodegradable" we mean that the restraining portion is degraded in the body of the patient, and therefore has a relatively short half-life, for example less than 72 hours, preferably less than 48 hours.

The inhibitor may alternatively, for example, be an antibody or part thereof which binds to the active drug, but not to the corresponding prodrug, or does so only to a more limited extent.

The production of antibodies to low molecular weight compounds such as cytotoxic drugs may be facilitated by the well known technique of haptenisation, in which the low molecular weight molecule is conjugated to a highly immunogenic protein, such as keyhole limpet haemocyanin or other carrier molecule. In situations where prodrug is converted to active drug by catalytic cleavage of a moiety of the prodrug, the production of an antibody capable of discriminating between prodrug and active drug is favoured since, in the prodrug, the non-active moiety can sterically inhibit binding of an anti-drug antibody to the thus hidden drug portion of the prodrug.

Alternatively, catalytic macromolecules can be exploited beneficially in the context of cytotoxic antimetabolite compounds for which normal metabolic components exist and which can be used to block the action of the antimetabolite. In this situation, the said "first compound" is a normal metabolite and the "second compound" is a derivative thereof which does not block the action of the cytotoxic antimetabolite. The catalytic macromolecule (for example a macromolecule-enzyme conjugate) can be used to degrade the metabolite at tumour sites whilst allowing the metabolite to protect the normal tissues. In this instance the macromolecule enzyme conjugate is first administered and allowed time to localise at tumour sites. An anti-enzyme antibody is administered say 24 hr after the enzyme conjugate and has the effect of reducing enzyme activity in the blood and normal tissues so that the subsequently co-administered antimetabolite and metabolite result in the metabolite protecting normal tissues whereas, in the tumour tissue, persisting enzyme inactivates the metabolite and thus exposes the tumour cells to the action of the antimetabolite. See WO 93/13805; in particular Figure 2 of WO 93/13805 illustrates the system in relation to CPG2 directed to the target cell and being used to remove folinic acid from the tumour site so that there is no longer inhibition of the action of trimetrexate (incorporated herein by reference).

Hence, any sufficiently non-toxic substance which in its native state is able to inhibit the effect of a cytotoxic agent may be converted into a substance which has less effect on said cytotoxic agent. A suitable compound is folinic acid. Folinic acid reverses the biological effect of the cytotoxic agent trimetrexate, for example, which acts on the enzyme dihydrofolate reductase. Folinic acid is deglutamated and rendered inactive against trimetrexate by the enzyme carboxypeptidase G2 and other deglutamating enzymes.

The drug trimetrexate (NSC 352122; 2,4-diamino-5-methyl-6-[3,4,5-trimethoxyanilinomethyl]quinazoline) acts by binding to dihydrofolate reductase; whereas methotrexate enters cells via the folate receptors, trimetrexate enters by alternative mechanism(s). The synthesis of trimetrexate is disclosed by Baker (1967) in *Design of site-directed irreversible enzyme inhibitors,* Wiley, New York, and by Elslager *et al* (1974) *Lectures in heterocyclic chemistry,* Vol. 2, pp 97/5-133 (Castle & Townsend, ed), Hetero Corp, Oren, Utah. Trimetrexate is generally available from US Biosciences, One Tower Bridge, 100 Front Street, Suite 400, West Conshohocken, PA 19428, USA.

Methotrexate resembles natural folates in having a terminal glutamic acid moiety which can be cleaved by carboxypeptidase G2, whereas trimetrexate is not susceptible to the action of this enzyme (Bagshawe (1985) *Clinical Radiol.* **36,** 545-551). The action of trimetrexate on colonic cancer cells *in vitro* can be enhanced by the addition to the culture medium of the folate degrading enzyme carboxypeptidase G2 (Searle *et al* (1990) *Biochemical Pharmacol.* **39,** 1787-1791.

The biological effect of both methotrexate and trimetrexate can be reversed by administering an end product of the reaction they block, or by a more readily available analogue known as folinic acid [5-formyl tetrahydrofolic acid]. Folinic acid is widely available, for example as Leucovorin from Cyanamid Inc, but also from Wellcome Inc, and Farmitalia. If folinic acid is given in sufficient dosage concurrently with methotrexate or trimetrexate their actions are blocked.

The same principle may be applied to other anti-cytotoxic agent substances. For example, thymidine blocks the effect of a cytotoxic agent, such as CB3717 and ICI D1694 (Jodrell *et al* 1991, *BJC* **64**, 833-8; Jones *et al* (1986) *J. Med. Chem.* **29,** 468-472), which acts on the enzyme thymidylate synthetase. Hence a thymidine degrading enzyme (such as dihydrothymine dehydrogenase, Shiotani & Weber 1981 *J. Biol. Chem.* **256,** 219-224) or thymidine kinase (Shiotani *et al* (1989) *Cancer Res.* **49,** 1090-1094) may be used to render the thymidine ineffective against the cytotoxic agent.

Similar considerations relate to other agents which interfere with the normal processes of nucleotide incorporation into DNA or RNA since these are potentially reversible by the normal metabolite which in turn can be degraded by an appropriate enzyme targeted to tumour sites.

For instance, it has been shown that the cytotoxic effects of the widely used cytotoxic 5-fluorouracil (available from Roche Products Inc) can be at least partly attenuated by uridine (Groeningen *et al (1989) J. Natl. Cancer Inst.* **81**, 157-162). Hence, conjugation of a macromolecule with a uridine degrading enzyme can be used in conjunction with 5-fluorouracil and uridine. Such a combination may be particularly relevant in colorectal and breast carcinoma for which 5-fluorouracil is one of the most effective cytotoxic agents. Such a combination of agents may be further combined with folinic acid which augments the cytotoxicity of 5-fluorouracil or additionally with thymidine and a thymidine inactivating enzyme.

The enzyme may be of human or non-human origin. It may not be necessary to use a conventional enzyme. Antibodies with catalytic capacity have been developed (Tramontano *et al Science* **234**, 1566-1570) and are known as 'abzymes'. These have the potential advantage of being able to be humanized to reduce their immunogenicity.

The bacterial enzymes carboxypeptidase Gl and G2 (CPG1 and CPG2) degrade folates including methotrexate by cleavage of the terminal glutamic acid. The actions of the two enzymes are thought to be the same. The following description of preferred aspects of the invention refers to CPG2 but is equally applicable to CPG1 and to any other enzymes acting on the same substrates, and to abzymes acting on the same substrates.

The isolation, purification and some of the properties of carboxypeptidase G2 from *Pseudomonas sp.* strain RS-16 have been disclosed by Sherwood *et al* (1984) *Eur. J. Biochem.* **148**, 447-453. The cloning of the gene encoding the said carboxypeptidase G2, its nucleotide sequence and its expression in E. *coli* have been disclosed by Minton *et al* (1984) *Gene* **31**, 31-38 and Minton *et al* (1983) *J. Bacteriol.* **156**, 1222-1227. CP2G2 is available from the Division of Biotechnology, Centre for Applied Microbiological Research, Porton Down, Salisbury, UK. Carboxy-peptidase G1 (CPG1) is disclosed by Chabner *et al* (1972) *Cancer Res.* **32**, 2114-2119.

Alternatively, the cytotoxic/drug may be converted into something less toxic by an agent (usually an enzyme) which is selectively located in the vascular compartment, as is described in WO 93/13806 (incorporated herein by reference). In particular the system is illustrated in Figure 4 of WO 93/13806 by reference to carboxypeptidase A being targeted to a target cell (such as a tumour) and converting Ala-Methotrexate to Methotrexate, and carboxypeptidase G-2 being restrained in the vasculature and inactivating Methotrexate by deglutamation.

A component may be required to avoid the constraints which could be imposed by the host immune response to foreign proteins. The nature of this component may change with the stage of development of methods of immunological control. Methods which can be used to overcome the host response to foreign protein are known in the art. A technique for reducing the immunogenicity of foreign proteins, applicable to macromolecule-enzyme conjugates and to inhibitory enzyme conjugates, is that of conjugation to forms of polyethylene glycol (Wilkinson *et al* (1987) J. *Immunol.* **139,** 326-331) or to low molecular weight dextrans (Mikolajezck *et al,* 1996 or Melton *et al,* 1987).

Alternatively, or additionally, the problem of immunogenicity may be overcome by administering immunosuppressors or immune tolerance inducing agents. Cyclosporin and FK506 are widely used drugs to achieve immunosuppression in tissue transplantation. Cyclosporin has been shown to delay host antibody response to foreign protein (Lederman *et al* (1988) *Br. J. Cancer* **58**, 562-566 and 654-657). Tolerance to foreign proteins when the host encounters the foreign problem for the first time after receiving an antibody directed at the CD4 epitope on lymphocytes has been disclosed (Waldman *et al* (1988) pp 16-30 in *Progress in Allergy* (Shizata & Woksman, Eds, New York). Further means to achieve this have been described elsewhere and may change as improvements occur in control of host antibody responses to foreign antigens. Catalytic antibodies (abzymes) may be 'humanized' to reduce or remove their immunogenicity.

A further aspect of the present invention provides a method of destroying target cells in a host, the method comprising administration to the host of the various components described above.

The components of the invention are administered in any suitable way, usually parenterally, for example intravenously, intraperitoneally or intravesically, in standard sterile, non-pyrogenic formulations of diluents and carriers. The blood concentration of the macromolecule may be measured every few hours. Typically, after 24 hours, the concentration of the macromolecule in the tumour is greater than its concentration in the blood.

An enzyme conjugated to polyethylene glycol or low molecular weight dextran might be expected to be of low immunogenicity. Such a macromolecule may be administered by slow intravenous infusion in a dosage sufficient to locate an optimal concentration of enzyme at tumour sites. After allowing time (usually 24h) for the conjugate to localise at tumour sites, an enzyme inhibitor may be administered by slow intravenous infusion. The inhibitor may be a monoclonal antibody directed at the conjugate, or it may be more specifically directed so as to inactivate the enzyme; in the latter case the antibody may be galactosylated to aid its rapid removal from the blood and to prevent it inactivating the enzyme at tumour sites. Alternatively, the inhibitor may be a small molecule which fits into the active site of the enzyme but is not catalysed by it (for example, suitable inhibitor systems are described in WO 97/20580, incorporated herein by reference). Such an inhibitor is administered at a dose level such that it inactivates enzyme in blood and normal tissues but only inactivates a small fraction of enzyme then located in tumours. In this way a great differentiation in enzyme concentration between normal and tumour tissues can be attained. When enzyme activity is no longer detectable in blood samples, a prodrug which is a substrate for the enzyme is given by repeated bolus injections or by infusion and continued until the enzyme concentration in the tumour falls below an effective value. The enzyme concentration in tumours can be monitored by gamma camera when a small component of the conjugate is conjugated to a suitable radioisotope such as ¹³¹Iodine using the technique of single photon emission computerised tomography (SPECT). Algorithms are well established which allow quantification of the enzyme at tumour sites. The values obtained compare closely with those obtained by HPLC on tumour biopsies.

In attempting to achieve eradication of cancers it may not be possible to avoid suppression of haemopoietic function (myelosuppression) although for a given effect on a tumour target it is expected to be much less with the system described herein. Similarly, for a given degree of myelosuppression a much greater tumour effect is expected. Growth factors or growth inhibitory factors acting on haematopoietic tissues may therefore be usefully employed in combination with the system described herein.

The system described herein may be used in conjunction with other forms of therapy. These include conventional cytotoxic agents, and use of multiple enzyme delivery to inactivate more than one metabolite.

Similarly, an enzyme delivered to tumour sites by a macromolecule may function both to activate a pro-drug and to inactivate a metabolite which protects normal tissues. Carboxypeptidase G2 inactivates folinic acid at tumour sites to leave the tumour cells unprotected against trimetrexate. The same tumour located enzyme can activate a benzoic acid pro-drug to form a cytotoxic mustard (as disclosed by Bagshawe (1989) *Brit. J. Cancer* 60, 275-281).

A further aspect of the invention provides the use of a catalytic macromolecule which does not specifically bind to a tumour antigen but which is capable, following administration to the vascular compartment of a mammal, of being taken up by a tumour in the mammal and is capable of converting a first compound into a second compound in the manufacture of a medicament for combatting a tumour in a patient, wherein the patient has been, is being or will be administered a first compound, and has been, is being or will be administered an inhibitor which, following administration to the said vascular compartment, reduces the level of the said catalytic conversion activity in the vascular compartment and normal (non-tumour) tissues.

The compounds and methods of the present invention will be discussed in the following Examples with specific references to cytotoxic therapy utilising the agent methotrexate, which is inactivated by the enzyme carboxypeptidase G2. The agent aminopterin is another powerful cytotoxic agent which is inactivated by the enzyme carboxypeptidase G2.

The invention will now be described in more detail by reference to the following Examples and Figures wherein:

Figure 1 shows the biodistribution of ¹²⁵I-[PEG-CPG2] in LS174T human colon cancers xenografted in nude mice.(see Example 9).

Figure 2(a). CPG2 conjugated to PEG 5000 mwt, average 32 PEG molecules per molecule of CPG2, 20 µg per mouse (nude mice bearing the human colon carcinoma LS174T) i.v. Figure shows percentage of injected dose per gram in tissues.

Figure 2(b). The corresponding tumour to normal tissue ratios.

Figure 3(a). CPG2 conjugated to PEG 2000 mwt, average 27-PEG molecules per molecule of CPG2, 25 enzyme units per mouse (nude mice bearing the human colon carcinoma LS174T) HPLC study.

Figure 3(b). The corresponding tumour to organ ratios. No clearance.

Figure 4(a). Biodistribution of CPG2 activity following use of galactosylated SB43 (clearing antibody). Mice (nude mice bearing the human colon carcinoma LS174T) were given 25 units per mouse i.v. of CPG2 conjugated to PEG 2000 mwt, average 22 moles PEG per molecule of CPG2, at time 0. Galactosylated monoclonal antibody SB43 was given 19, 20 and 21 hrs later by IP route. The mice were killed at 24 h post CPG2.

Figure 4(b). Tumour to organ ratios with above protocol.

Figure 5. CPG2 activity in LS174T tumours comparing 20 units of CPG2 conjugated to PEG 5000 (MDEPT) or to monoclonal antibody A5B7-(Fab')₂ conjugated to CPG2 (ADEPT).

Figure 6. Nude mice bearing LS174T tumours were given 25 enzyme units of CPG2-PEG (22 PEG molecules, PEG 5000) followed at 20 hr, 23 h, 26 h by galactosylated SB43. At 48 h post CPG2-PEG they received 25 mg/kg of a phenol prodrug (4-[N,N-bis(2-chloroethyl)amino]-phenoxycarbonyl-L-glutamic acid) intraperiotoneally, repeated to a total of 3 doses at 1 hr intervals.

Figure 7. The biodistribution of native CPG2 and PEG-CPG2 at 72 hours is illustrated. CPG2 activity in tumour (LS174T xenografted into nude mice) is shown. Mice were injected with 25 units of either CPG2-PEG or CPG2-native.

Figure 8. The biodistribution of ¹²⁵I-labelled native CPG2 and PEG-CPG2 in LS174T xenografted nude mice is shown.

### Example 1: A therapeutic system using Ala-methotrexate carboxypeptidases A and G2

The widely used agent methotrexate is a folic acid antagonist and its action is to block the conversion of folic acid, a dietary factor, to its reduced form 5-methyltetrahydrofolic acid (folinic acid, citrovorum factor) by the enzyme dihydrofolate reductase. Folinic acid is used in one carbon transfer in the synthesis of DNA. In the absence of folinic acid, cell reproduction is blocked in S phase and cell death follows. Methotrexate is used in the treatment of a wide range of malignant diseases. It also causes cell death in normal cell renewal tissues via the mechanisms already outlined. The magnitude of its effects is largely a function of the duration of tissue exposure to the drug, the longer the duration the greater the toxic effect. Susceptibility to the action of methotrexate is thought to result from polyglutamation of the drug which, by delaying its breakdown within and its excretion from the cell, favours prolonged action. The effect of methotrexate can be by-passed by folinic acid, generally given in the form of 5-formyl tetrahydrofolic acid. Carefully timed and dose-controlled administration of methotrexate with folinic acid has been found advantageous over the use of methotrexate alone in the treatment of some cancers. Thus large doses of methotrexate are commonly followed after 12-24 hours by folinic acid 'rescue'. Similarly, administration of low dose methotrexate on alternate days and folinic acid on each succeeding day has proved to he a successful and low toxicity treatment for many patients with some forms of trophoblastic tumour (Bagshawe *et al (1989) Brit. J. Ob. Gynae* **96,** 795-802).

It has been shown (Kuefner *et al (1989) Biochemistry* **28,** 2288-2297) that when methotrexate is modified by introducing an alanine moiety via an amide linkage to the α carboxyl of methotrexate the resulting compound is effectively excluded from cells and the toxicity of the alanine form is 50-100 fold less than that of native methotrexate in target cells *in vitro.* The alanine moiety is cleaved by the action of the enzyme carboxypeptidase A leaving native methotrexate. Alanine methotrexate is not metabolised by the enzyme carboxypeptidase G2. Carboxypeptidase G2 degrades methotrexate and natural folates by cleavage of the glutamic acid moiety.

In the present example, the catalytic macromolecule of the system of the present invention comprises a macromolecule such as polyethylene glycol, a dextran or an immunoglobulin conjugated to carboxypeptidase A. Of course, a carboxypeptidase other than carboxypeptidase A but with the same substrate specificity may be used in place of carboxypeptidase A.

Carboxypeptidase A is available from bovine and bacterial sources (for example from Calbiochem, Nottingham, UK) and is also present in human pancreas. The enzyme hydrolyses oligopeptides from the C terminal end of polypeptide chains, or from other compounds containing conjugated amino acids with a free carboxyl group. Carboxypeptidase A has a preference for aromatic residues. It is normally formed from mammalian sources by trypsinization of a complex assembly of three subunits produced by the pancreas.

The CPA is conjugated to PEG or dextran by conventional techniques, for example as in WO 90/13540.

The "first compound" is alanine methotrexate (Kuefner *et al* 1989) which is the prodrug from which the active drug methotrexate (the "second compound") is generated by the action of carboxypeptidase A.

The "inhibitor" is carboxypeptidase G2 conjugated to a macromolecular structure such as dextran. The purpose of this macromolecule is to confine CPG2 activity to the vascular compartment.

For example, CPG2 may be coupled to soluble dextrans (Lomodex 40, Lomodex 70, Dextraven 110 and Dextraven 150, all trade marks; Fisons, Loughborough, Leics., UK) according to the method of Melton *et al* (1987). A volume of dextran preparation containing 1 g of dextran in 0.9% NaCl was diluted to 100 ml with 0.9% NaCl and reacted with cyanogen bromide (CNBr; Sigma, Poole, Dorset, UK). CNBr (0.5 g) was used for activating the 40- and 70,000 dalton dextrans and 0.4 g for the higher molecular weight dextrans. This reduction was necessary to prevent precipitation of the 110,000 and 150,000 dalton dextrans. The reaction mixture was vigorously stirred at room temperature and maintained at pH 10.7 ± 0.1 units in a pH-stat (Radiometer, Copenhagen, Denmark) by addition of 2 M NaOH. The CNBr was added as a finely divided powder in two equal portions at an interval of 20 min; the second portion was allowed to react until the pH of the reaction mixture was stable at 10.7; the pH was then adjusted to 9.0 and the mixture dialysed against running water for 2 hr at 4°C. The pH was brought back to 9.0 with 1 M NaOH and 1 ml enzyme solution (1265 U; 2.3 mg) in 0.1 M Tris-HCl buffer, pH 7.3, was added. The mixture was reacted overnight at 4°C after which 0.25 g glycine was added to block excess reactive sites. The mixture was stirred for a further 30 min and then concentrated to a volume of 40 ml in a model 202 concentrator using a PM10 ultrafiltration membrane (Amicon, Stonehouse, UK). The mixture (40 ml) was then chromatographed on a 1.3 litre bed volume of Sephadex G150 in a 4.4 x 87 cm column (Pharmacia, Uppsala, Sweden) and eluted with 0.05 M potassium phosphate buffer, pH 7.0. Fractions (10 ml) were collected and assayed for enzyme activity; carbohydrate content was determined by the phenol-sulphuric acid method (M. Dubois *et al* (1956) *Analyt. Chem.* **28**, 350) using dextran-70 as standard in the range 0-100 µg/ml (Sephadex is a trade mark).

The peak fractions were pooled and concentrated to a volume of 10-12 ml as before. Enzyme activity and carbohydrate content were determined and protein content measured by the Coomassie blue method (M.M. Bradford (1976) *Analyt. Biochem.* **72,** 248) using bovine serum albumin fraction V as standard in the range 0-100 µg/ml. The concentrated material was filter sterilised (Millipore "Millex GS", 0.22 µm pore size) and stored at -20°C. Millex GS is a trade mark.

The macromolecule-carboxypeptidase A conjugate, if comprising enzyme of bovine origin, would be immunogenic. Similarly, a carboxypeptidase G2 macromolecule conjugate would also be immunogenic since CPG2 is bacterial in origin. It may be desirable, therefore, to reduce their immunogenicity or to employ means to induce immunosuppression or immune tolerance.

### Example 2: Method of use

Administration of a component capable of overcoming the host response to foreign protein may be started 48 hours before administration of the catalytic macromolecule. Initial tests may be performed to exclude as far as possible abnormal reaction by the patient to any of the protein components. The CPA conjugate is given intravenously, preferably by slow infusion, typically over 2 hours. Maximal tumour concentration of the CPA conjugate is achieved several hours later but at this time there are still high levels of CPA activity in plasma. It is desirable to eliminate this activity as far as possible before administering the prodrug. This elimination process is achieved by administration of a component (such as an anti-CPA antibody, which may be galactosylated) capable of achieving accelerated clearance or inactivation of CPA from non-tumour sites. This component is administered intravenously over several hours, typically 6-24 hours, or until enzyme is no longer detectable in plasma and may be infused at low concentration throughout the period of pro-drug administration. During this time enzyme in extracellular fluid diffuses back into the plasma as the plasma level of the enzyme falls. Tests for enzyme activity from plasma are continued for a period typically 8-24 hours to ensure that plasma CPA activity is not detectable. More of said component which eliminates the CPA activity is given if necessary. Alternative methods of accelerated clearance have been described.

The CPG conjugate is then administered either by a series of bolus injections or by slow infusion, and it is preferred that the prodrug is administered simultaneously.

Pro-drug may be given as a series of bolus injections or by continuous infusion. Administration of the prodrug and CPG conjugate will normally continue for about 4-7 days.

At about 7-10 days after administration of the CPA conjugate, it is desirable to review enzyme activity at tumour sites. Administration of the component capable of overcoming the host response to foreign protein is continued, the prodrug is discontinued, and the CPG conjugate may be discontinued.

The CPA conjugate is reinfused as previously, followed by the component which eliminates the plasma CPA activity as previously. Similar procedures to those previously described are followed before recommencing the prodrug.

The cycle may be repeated. Limiting factors will be toxicity attributable to alanine methotrexate or the development of host antibodies to any of the foreign proteins employed.

### Example 3: Use of quinazoline antifolates

A similar system to that disclosed in Examples 1 and 2 can be used with at least some members of a series quinazoline antifolates which have been described (Jones *et al* (1986) *J. Med. Chem.* **29,** 468-472; Jodrell *et al* (1991) *Brit. J. Cancer* **64,** 833-8; Harrap *et al* (1989) *Advances in enzyme regulation* **29,** 161; Jackman *et al* (1991) *Advances Enzyme Regulat.* **31,** 13; Jodrell *et al* (1990) *Proc. Am. Assoc. Cancer Res.* **31,** 341. These agents differ from natural folates and methotrexate with respect to substitution for instance at the N¹⁰ position and in the benzoyl ring but like natural folates and methotrexate have a terminal glutamate moiety linked to the benzoyl ring. Therefore at least some of the drugs in this series are inactivated by a peptide substitution such as an alanine in the a position of the glutamate, and that such alanine- or other peptide-substituted derivatives are synthesized using the methodology described by Kuefner *et al loc. cit.* or variations thereof known in the art. Similarly, such quinazoline antifolates are deglutamated, and therefore inactivated, by carboxypeptidase G2 or a similar enzyme. These compounds are of particular interest because they act by inhibition of thymidylate synthetase. The chemical application of such drugs may be greatly extended by being administered in pro-drug form, converted to the active compound by carboxypeptidase A and the active drug in plasma degraded by carboxypeptide G2.

### Example 4: Production of antibodies discriminating between active drug and pro-drug

In order to raise an antibody that would recognise the active drug (II) but not the pro-drug (I), a compound was synthesised which represented the region of greatest divergence between the two molecules (I) and (II). This region corresponds to the acid portion of the benzoic acid mustard drug (II). Since benzoic acid itself is not large enough to be immunogenic; it was considered that the most effective method of raising antibodies specific for the acid region would be to inoculate animals with a benzoic acid analogue that had been previously conjugated to keyhole limpet haemocyanin (KLH). A compound was synthesised that consisted of an L-lysine amino acid linked through an amide bond to aminobenzoic acid (VII). Then (VII) was conjugated to KLH by conventional methods using the ε-NH₂ groups on the lysine portion of the molecule, to produce the specific immunogen (VIII), all as described in WO 93/13806.

### Example 5: Production of a monoclonal antibody reactive against carboxypeptidase G2

A monoclonal antibody raised against carboxypeptidase G2 is used for clearance and inactivation of residual enzyme activity at non-tumour sites.

The monoclonal antibody was made in the following way. Balb/C mice (6-8 weeks old) were immunised with 50 µg CPG2 i.p. in incomplete Freund's adjuvant followed by two injections of CPG2 in complete Freund's adjuvant (50 µg CPG2 each, i.p.) at monthly intervals and with two daily injections (50 µg and 100 µg in PBS, i.v.) 2 days before fusion. Immune spleen cells were fused with non-immunoglobulin secreting SP2/0 myeloma cells according to the hybridoma procedures of Köhler and Milstein (1975).

The presence of anti-CPG2 antibodies was detected by a solid-phase indirect radioimmunoassay. A 1 µg ml⁻¹ solution of CPG2 in 0.05 M phosphate buffer was placed in polyvinyl microtitre plates (100 ng per well), allowed to dry, fixed with methanol and washed with PBS buffer containing a 0.05% Tween and 0.1 % bovine serum albumin. Supernatant or purified antibody samples were diluted in PBS and incubated in the CPG2 coated microtitre plates (100 µl per well) at 37°C for 4 h and then for 1 h with ¹²⁵I-labelled rabbit anti-mouse IgG. The wells were washed three times with PBS-Tween buffer between each stage and after final washing individual wells were cut and counted in a gamma counter.

### Example 6: Reduction of residual enzyme activity at non-tumour sites

It is desirable to inactivate the enzymatic portion of the enzyme-macromolecule conjugate at non-tumour sites, but not at the tumour. One method of achieving this effect is to administer to the patient being treated using the compounds of the invention antibodies raised against the enzyme portion which have been conjugated with galactose residues.

A monoclonal antibody directed at CPG2 inactivates the enzyme. To prevent the antibody inactivating the enzymes at tumour sites, additional galactose residues are conjugated to it so that it can still inactivate enzyme in plasma when it is given by intravenous route but the inactivating antibody is rapidly removed from the plasma by galactose receptors on hepatocytes.

The galactosylated anti-CPG2 monoclonal antibody is given to eliminate enzymic activity in plasma and then to give an amount of the non-galactosylated anti-CPG2 monoclonal antibody to inactivate residual enzyme activity in other non-tumour tissues.

The monoclonal antibody is galactosylated using the following protocol. A stock solution of the activated derivative was made up as follows. Cyanomethyl 2,3,4,6-tetra-O-acetyl-1-thio-β-D-galactopyranoside (Sigma C-4141) [400 mg] in anhydrous methanol (10 ml) was treated with 5.4 mg of sodium methoxide in 1 ml of anhydrous methanol at room temperature for 48 hours. The mixture was kept in a 25 ml Quickfit conical flask fitted with a slightly greased stopper.

A stock solution of monoclonal antibody (1.3 mg/ml) is prepared in 0.25 M sodium borate buffer, pH 8.5. Aliquots of the required amount of activated galactosyl derivative (80, 40, 20, 10 µl) are dispensed into 3 ml glass ampoules and evaporated to a glassy residue in a stream of nitrogen. A solution of the antibody (200 µg) is added mixed until the residue is dissolved. After 2 hours at room temperature, the solution is dialysed against three changes of PBS.

The preparations are scaled up by taking multiples of the volumes mentioned above.

### Example 7: Cytotoxic therapy with trimetrexate and folinic acid rescue

The present invention may provide a means to allow more continuous anti-folate action at tumour sites whilst protecting normal tissues from anti-folate effects, as in WO 93/13805.

The first step is to initiate immunosuppressive or immune tolerance inducing agents and this will normally occur not less than 2 days before the compound of the present invention is administered. If the catalytic macromolecule is non-immunogenic this step is omitted.

A macromolecule-CPG2 conjugate is given by intravenous or other appropriate route. After several hours has been allowed for the conjugate to localise at tumour sites an antibody is given. This may be directed at the active site on the enzyme in which case additional galactose residues are attached to the antibody to ensure its rapid clearance via hepatocyte galactose receptors. Alternative mechanisms for rapid clearance of the conjugate from non-tumour sites have been described above. When enzyme levels have fallen to very low or undetectable levels in plasma, trimetrexate is given by bolus or by continuous infusions with the aim of maintaining a constant plasma concentration. Concurrently with the trimetrexate, folinic acid is given at a dose level sufficient to protect the normal tissues from trimetrexate toxicity. Folinic acid reaching tumour sites where CPG2 is located is deglutamated, as is folic acid, and rendered inactive before it can enter cells and protect them from trimetrexate. In this way normal tissues may be protected by folinic acid from the action of trimetrexate whereas the protective molecule is rapidly degraded at tumour sites. Other antifolate drugs which, like trimetrexate are not degraded by CPG2, may be substituted for trimetrexate.

### Example 8: Cytotoxic therapy with thymidylate synthetase inhibitors and thymidine rescue

The same principle may be applied to other anti-metabolite therapies. For instance, considerable effort has been directed in recent years to the development of agents that block the key enzyme thymidylate synthetase such as CB3717 and ICI D1694 (Jodrell *et al* 1991, *BJC* **64,** 833-8). Such agents are likely to suffer the same limitations as conventional cytotoxic agents in that they also block thymidylate synthetase in normal cells. Their action is reversible by thymidine. Thymidine administration may therefore be used to protect normal tissues from thymidylate synthetase inhibitors whilst the protective effect of the thymidine and endogenous thymidine may be limited at tumour sites by a thymidine degrading enzyme, such as dihydrothymine dehydrogenase, or thymidine kinase which would inhibit entry of thymidine into the tumour cells. The thymidine inhibitor enzyme or agent is delivered by the non-specific macromolecule, in accordance with the invention.

Dihydrothymine dehydrogenase can be obtained from normal human lymphocytes (Shiotani & Weber 1989 *Cancer Res.* **49,** 1090-1094) and therefore has the advantage that a macromolecule-enzyme conjugate of low immunogenicity may be produced by its conjugation to a non-immunogenic macromolecule.

### Example 9: Tumour take up of PEG-enzyme conjugate and of non-tumour specific IgG-enzyme conjugate

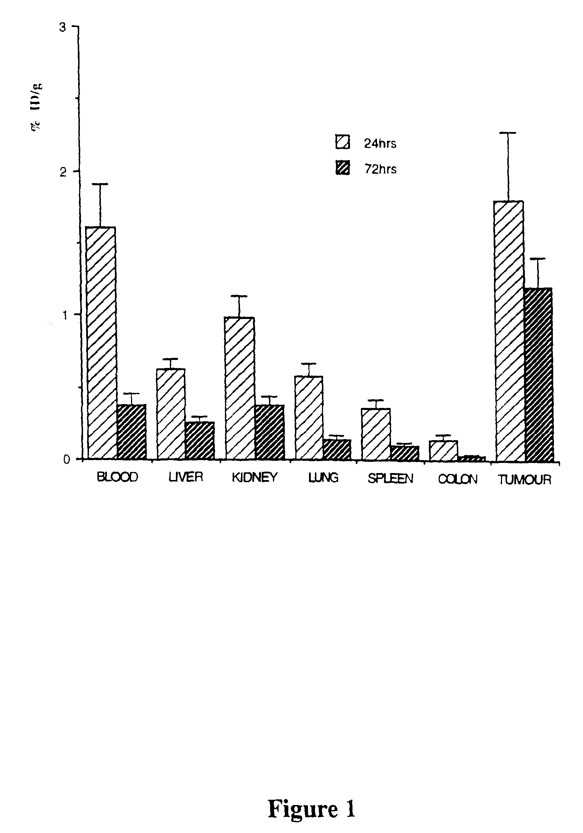

In LS174T human colon cancers xenografted in nude mice, 1.81% of administered dose of a ¹²⁵I-polyethylene glycol-carboxypeptidase G2 was found per gram of tumour at 24h and 1.2% was still present at 72h (see Fig 1). Native ¹²⁵I-CPG2 gave comparable values of 0.35% and 0.09%. Comparison of tissue distribution of ¹²⁵I anti-CEA monoclonal antibody conjugated to carboxypeptidase G2 in LS174T (CEA positive) and CC3 (CEA negative) xenografts gave the following results. At 24h after intravenous injection, LS174T contained 1.4%, whereas CC3 contained 0.9% of the injected dose per gram of tumour. At 48h, LS174T had 1.2%, whereas CC3 had 0.7% and, at 7 days, LS174T had 0.35%, whereas CC3 had 0.3%. Thus, the amount of conjugate retained in the non-CEA producing tumour was more than half that retained in the CEA producing tumour (S.K. Sharma, Thesis for PhD examination, London University, 1991).

It has further been shown (S.K. Sharma thesis), over a wide range of dosages of an antibody enzyme conjugate (20-300µg per mouse), that the percentage of injected dose per gram of tumour remained constant within experimental limits (1.57-1.77%).

Thus, the less efficient delivery of enzyme to tumour by non-specific macromolecule, compared with a tumour specific antibody, can be compensated by an increase in administered dosage of about 2-fold.

A further investigation of the distribution of CPG2 conjugated to PEG in nude mice bearing the human colon carcinoma LS174T was undertaken.

### Materials and Methods

Methoxypolyethylene glycol-p-nitrophenol carbonate of mol. wt. 5000 was obtained from Sigma. Methoxypolyethylene glycol-p-nitrophenol carbonate of mol. wt. 2000 was synthesised following the method described (Veronese, F.M., Largajolli, R., Boccu, E., Benassi, C.A. and Schiavon, O. (1985) Surface modification of proteins - Activation of monomethoxy-polyethylene glycols by phenyl chloroformates and modification of ribonuclease and superoxide dismutase. *App. Biochem.* Biotech. **11,** 141-152). 4-[*N,*N-bis(2-chloroethyl)amino]-phenoxycarbonyl-L-glutamic acid (phenol mustard prodrug) was synthesised according to the published procedure and its structure is shown in the figure (Dowell, R., Springer, C.J., Davies, D.H., Hadley, E.M., Burke, P.J., Boyle, F.T., Melton, R.G., Connors, T.A., Blakey, D.C. and Mauger, A.B. (1996). New mustard prodrugs for antibody-directed enzyme prodrug therapy (ADEPT): Alternative to the amide link. *J. Med. Chem.* **39,** 1100-1105). (This prodrug is described in WO 94/02450.)

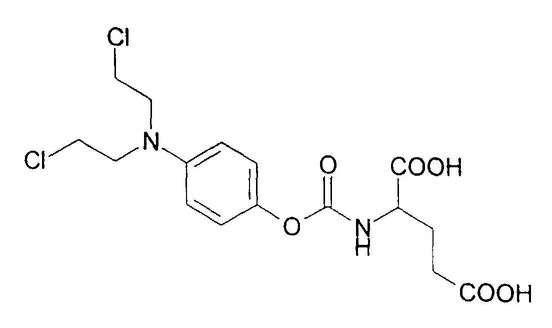

### Covalent attachment of PEG to CPG2

Either 516 mg of methoxypolyethylene glycol p-nitrophenyl carbonate of mwt 5000 or 206.4 mg of methoxypolyethylene glycol p-nitrophenyl carbonate of mwt 2000 was added to 38.13 mg of CPG2 in 6.7 ml of 0.2 M sodium phosphate buffer pH 7.2 (Veronese, F.M., Largajolli, R., Boccu, E., Benassi, C.A. and Schiavon, O. (1985) Surface modification of proteins - Activation of monomethoxy-polyethylene glycols by phenyl chloroformates and modification of ribonuclease and superoxide dismutase. *App. Biochem. Biotech.* **11,** 141-152). After 1 hr at room temperature unreacted PEG was removed in an ultrafiltration cell (Amicon) using an XM-50 membrane and 0.1 M sodium phosphate buffer, pH 7.2 as the dialysing solution. Polyethylene glycol modified CPG2 was separated from unmodified protein by FPLC gel filtration using a superose S/12 HR column (Pharmacia) equilibrated with 0.1 M sodium phosphate buffer pH 7.2 and eluted with the same buffer.

### Animal Studies

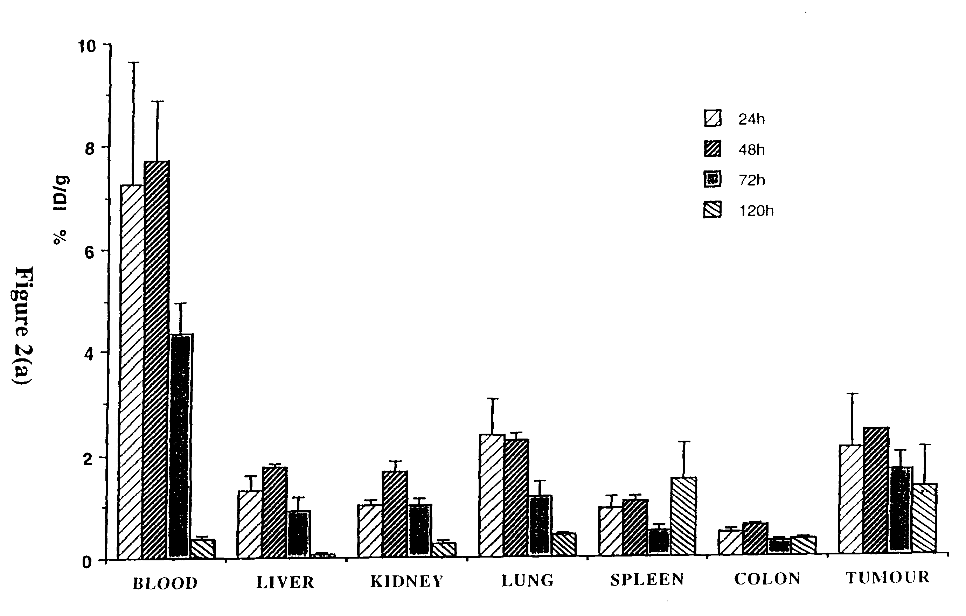

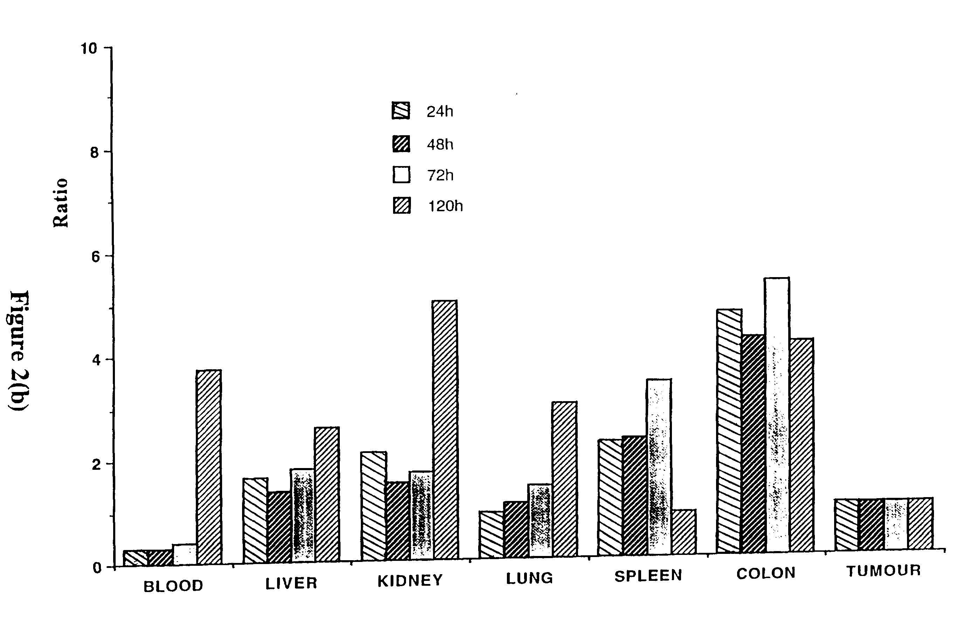

The following figures (Figures 2 to 6) illustrate the biodistribution of carboxypeptidase G2 conjugated to PEG in nude mice bearing the human colon carcinoma LS174T. The mice were injected intravenously with the specified amount of enzyme (measured as enzyme units) using 4 mice per time point. Mice were killed at 24 h, 48 h, and 72 h after injection, in some cases at 96 h and 120 h. CPG2 activity in plasma was measured by spectrophotometric assay using methotrexate as the substrate. CPG2 activity in tumour and other tissues was measured by HPLC using methotrexate as the substrate.

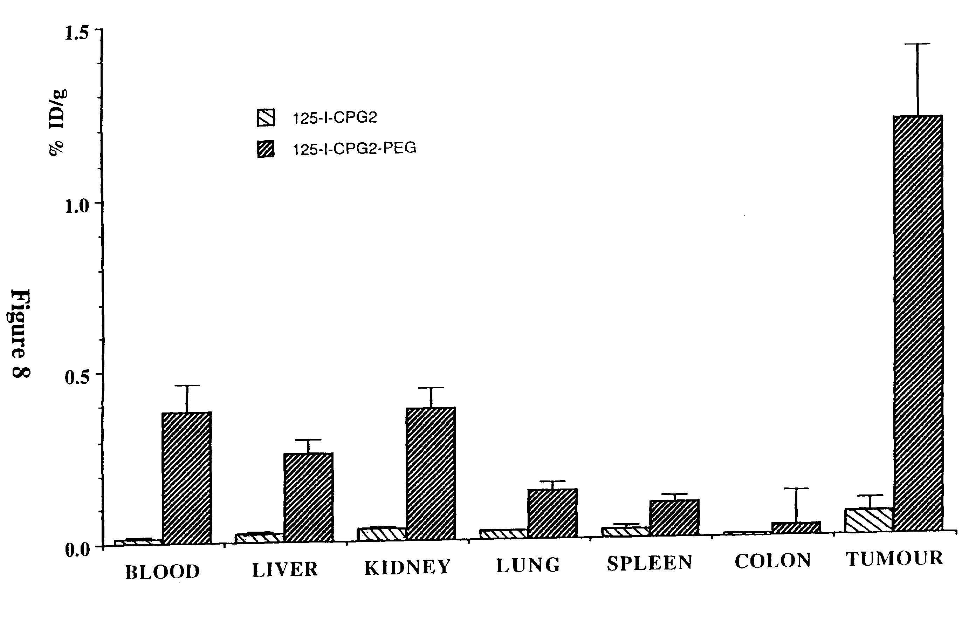

Unconjugated CPG2 clears rapidly from blood tumour and normal tissues. The biodistribution of native CPG2 and PEG-CPG2 at 72 hours is illustrated in Figures 7 and 8.

Figure 2(a). CPG2 conjugated to PEG 5000 mwt, average 32 PEG molecules per molecule of CPG2, 20 µg per mouse. Figure shows percentage of injected dose per gram in tissues.

Figure 2(b). The corresponding tumour to normal tissue ratios.

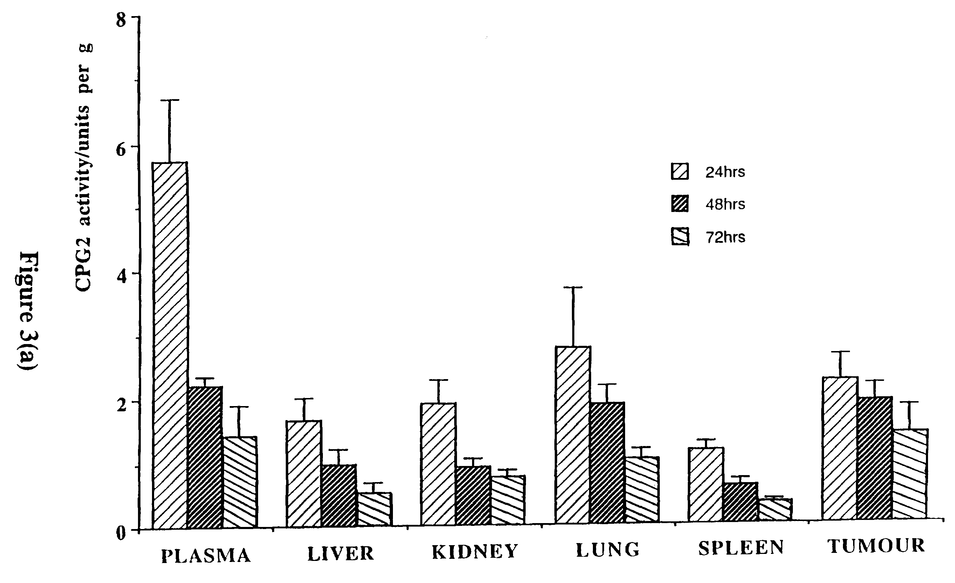

Figure 3(a). CPG2 conjugated to PEG 2000 mwt, average 27-PEG molecules per molecule of CPG2, 25 enzyme units per mouse.

Figure 3(b). The corresponding tumour to organ ratios.

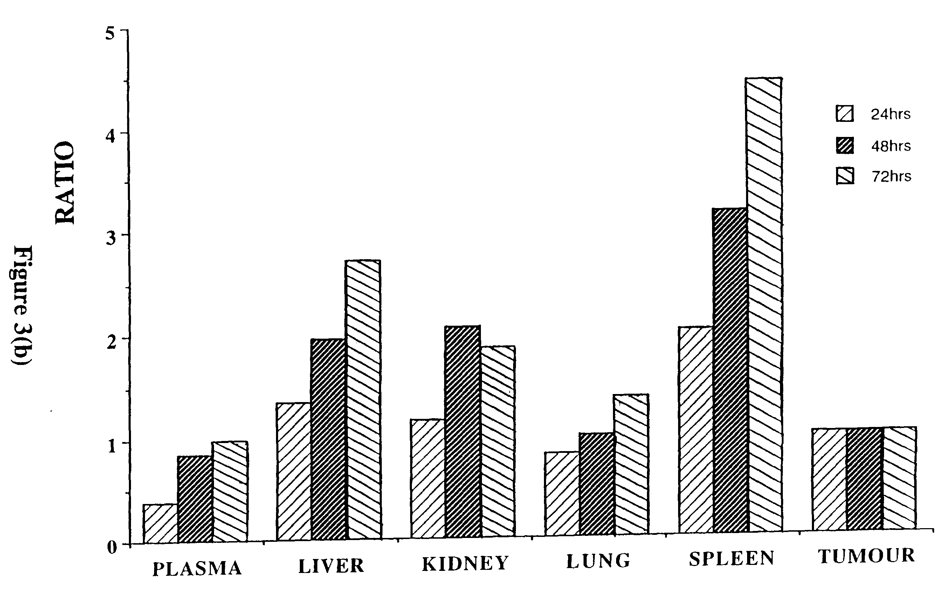

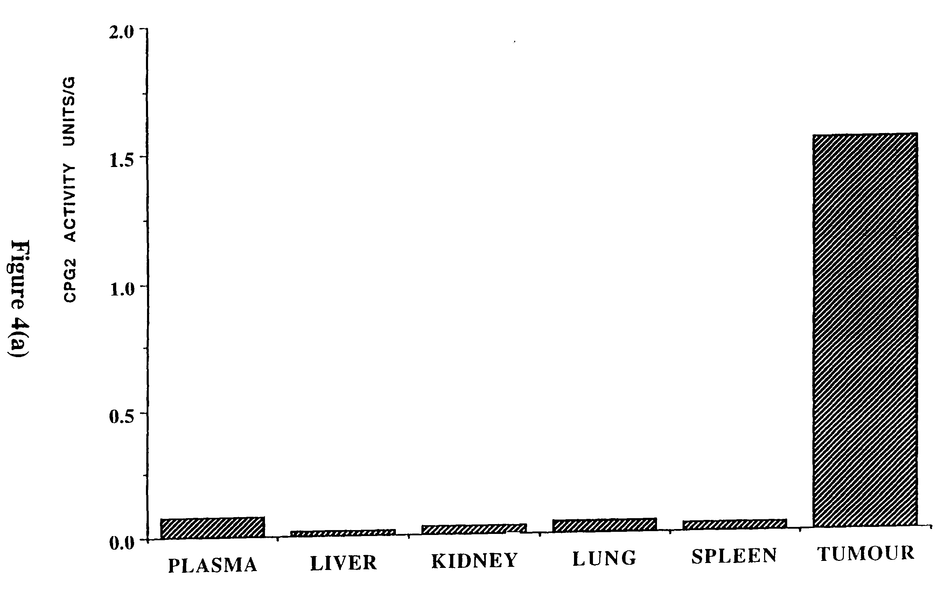

Figure 4(a). Biodistribution of CPG2 activity following use of galactosylated SB43 (clearing antibody). Mice were given CPG2 conjugated to PEG 2000 mwt, average 22 mols PEG per molecule of CPG2, at time 0. Galactosylated monoclonal antibody SB43 was given 19, 20 and 21 hrs later by IP route. The mice were killed at 24 h post CPG2.

Figure 4(b). Tumour to organ ratios with above protocol.

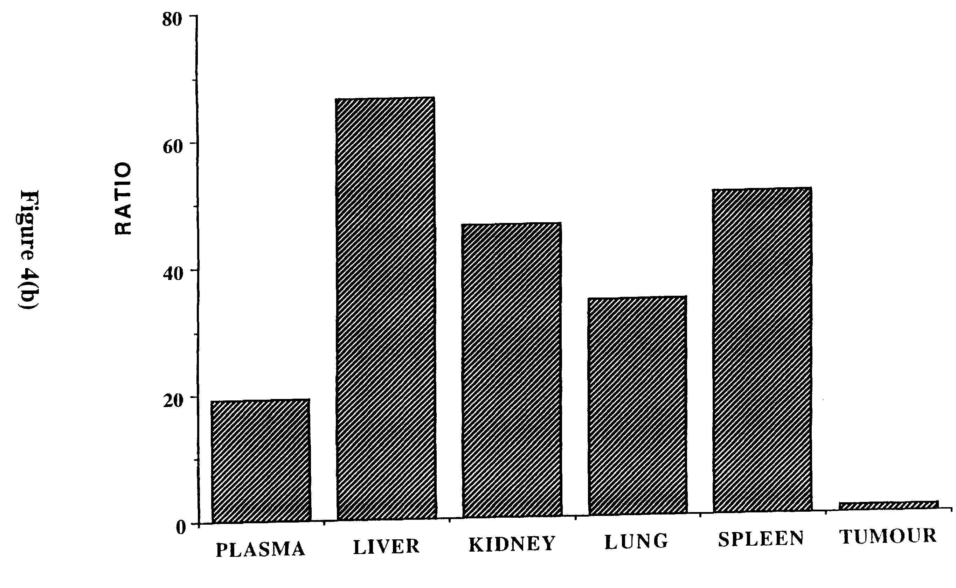

Figure 5. CPG2 activity in LS174T tumours comparing 20 units of CPG2 conjugated to PEG 5000 or to monoclonal antibody A5B7-(Fab')₂ conjugated to CPG2.

Figure 6. Nude mice bearing LS174T tumours were given 25 enzyme units of CPG2-PEG (22 PEG molecules, PEG 5000) followed at 20 hr, 23 h, 26 h by galactosylated SB43. At 48 h post CPG2-PEG they received 25 mg/kg of a phenol prodrug intraperiotoneally, repeated to a total of 3 doses at 1 hr intervals.

In relation to Figure 4(a) and 4(b) these show good tumour to normal tissue concentration and ratios following use of the clearing antibody. However, it is not possible to administer the clearing antibody as efficiently in the mouse as in the human patient.

At the therapeutic level in the LS174T tumour model I have shown significant growth delay compared to untreated controls when PEG 5000 at 22 PEGs per CPG2 are used with the and CPG2 clearance antibody SB43 and the phenol prodrug.

I have investigated PEG (mol wt 5000) in the range of 13-32 PEG molecules per enzyme molecule (CPG2, mol wt 83,000); the higher the molecular weight the higher and more prolonged is the plasma concentration of enzyme achieved. The higher the plasma concentration the higher tumour concentration in the tumour model LS174T. Since one objective is to get as much enzyme into the tumour as possible and prolong the period for which an effective concentration of enzyme is sustained in the tumour the conjugate giving the highest concentration of enzyme in blood is preferred. However, the choice of conjugate also has to take account of the need to clear enzyme from blood after the desired concentration of enzyme has been achieved in the tumour(s) and before prodrug is given. A conjugate which is difficult to clear from blood would be disadvantageous so that compromise between these two requirements may be necessary. The downside is that the high and persistent plasma concentration of PEG (5000) 32-CPG2 indicates that more clearing antibody should be used to lower the concentration of active CPG2 in the plasma.

In comparing PEG 5000 with PEG 2000, my studies show that tumour concentrations of enzyme are similar but plasma levels with PEG 2000 are lower. Thus, PEG with a molecular weight of 2000 in the range 20 to 40 PEG molecules per molecule of enzyme is preferred, giving a total molecular weight in the range of around 130-160 kDal, but associated water of hydration gives a molecular size equivalent to molecules of much greater weight.

### Example 10: PEG-NQO2 conjugation data

NQO2 is a much smaller enzyme (mol. wt. 24000) than CPG2 (mol. wt. 80000). In order to ensure that NQO2 is not readily excreted by the kidneys after intravenous administration and thus fail to localise in tumours, it is desirable to ensure that the molecular weight of the conjugate is > 70000. This was achieved by derivitisation and conjugation to polyethylene glycol mol. wt. 5000 (Peg 5000). Attachment of 14 molecules or more of Peg 5000 to each molecule of NQO2 resulted in a conjugate of estimated molecular weight of 90000.

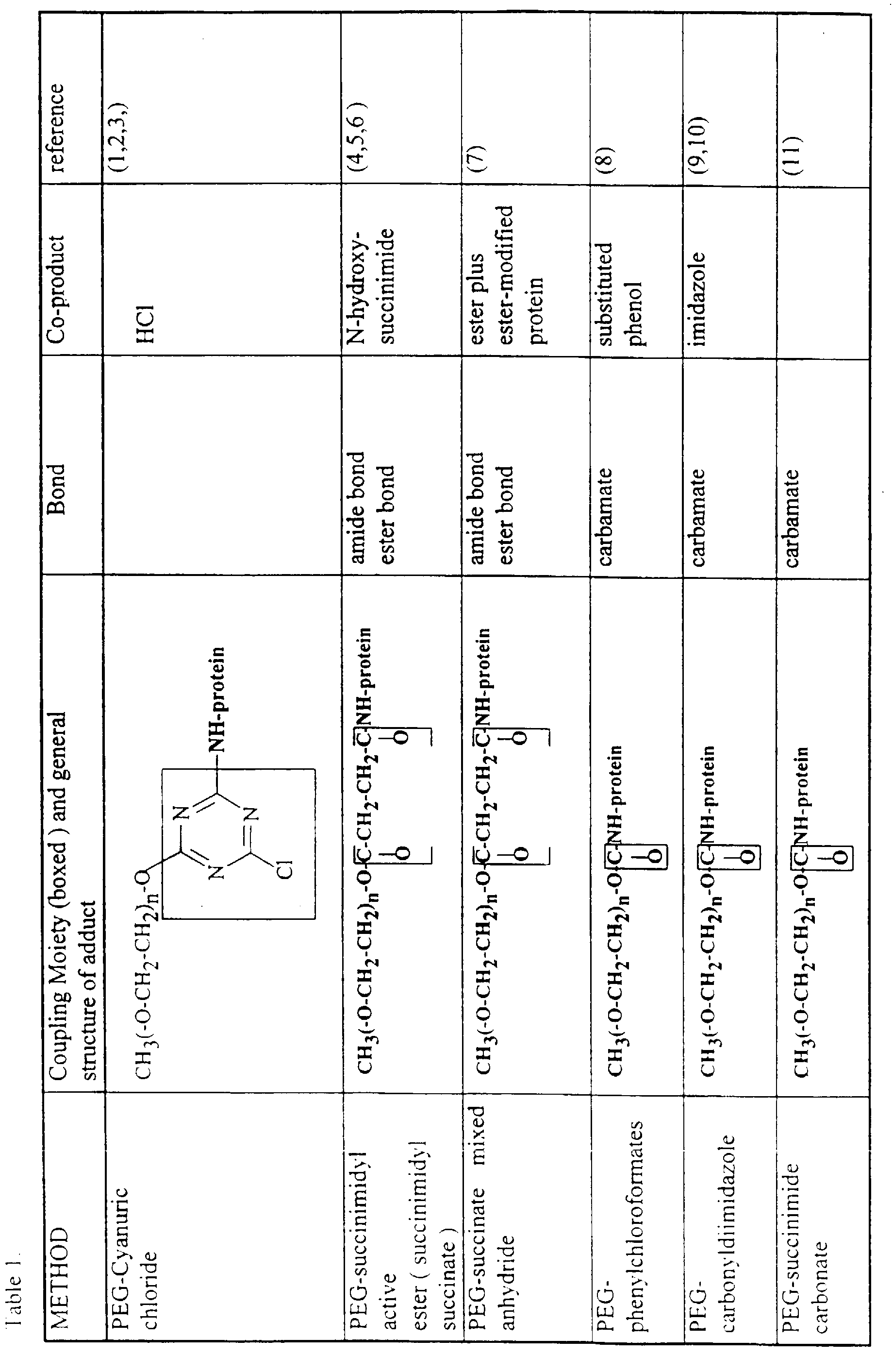

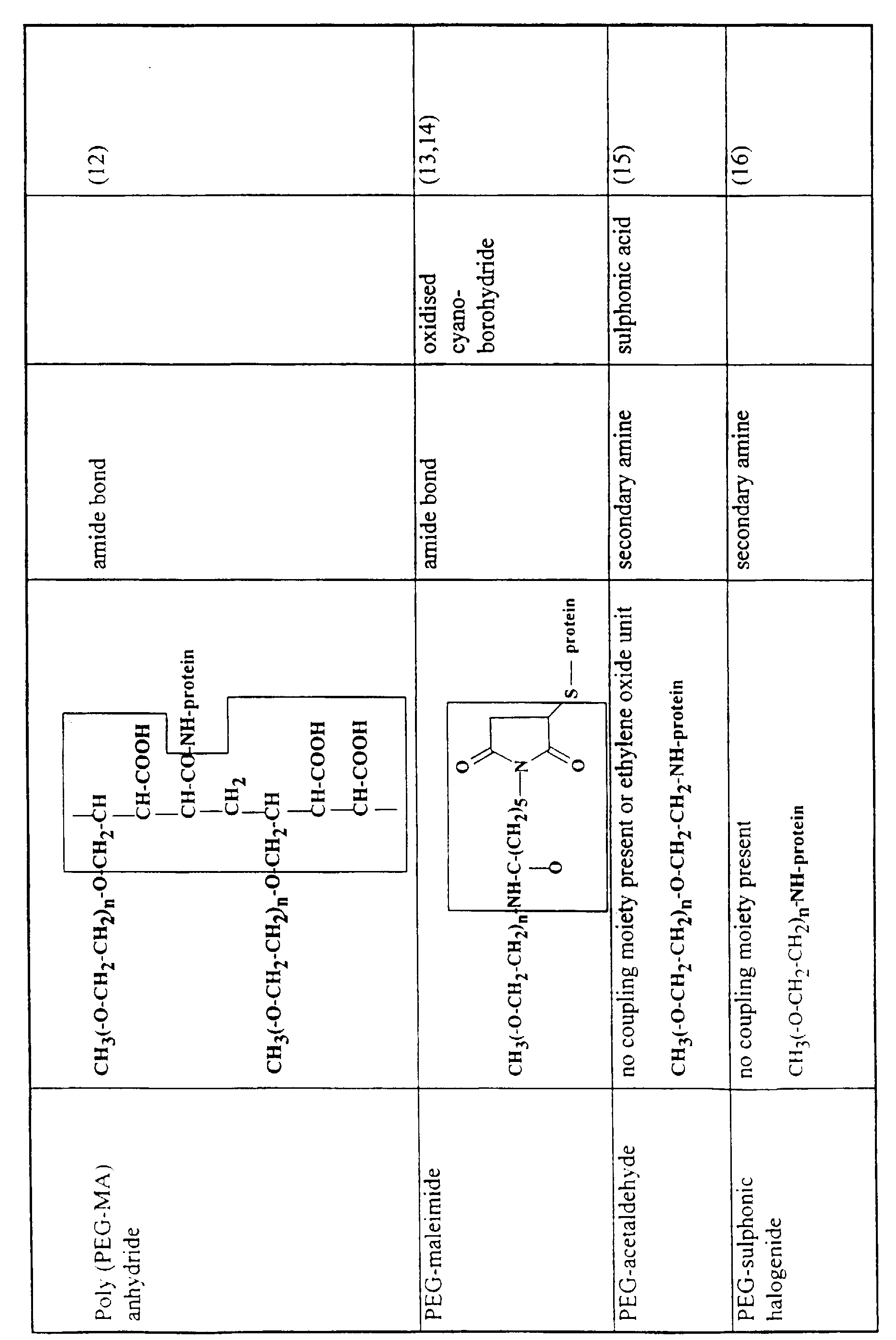

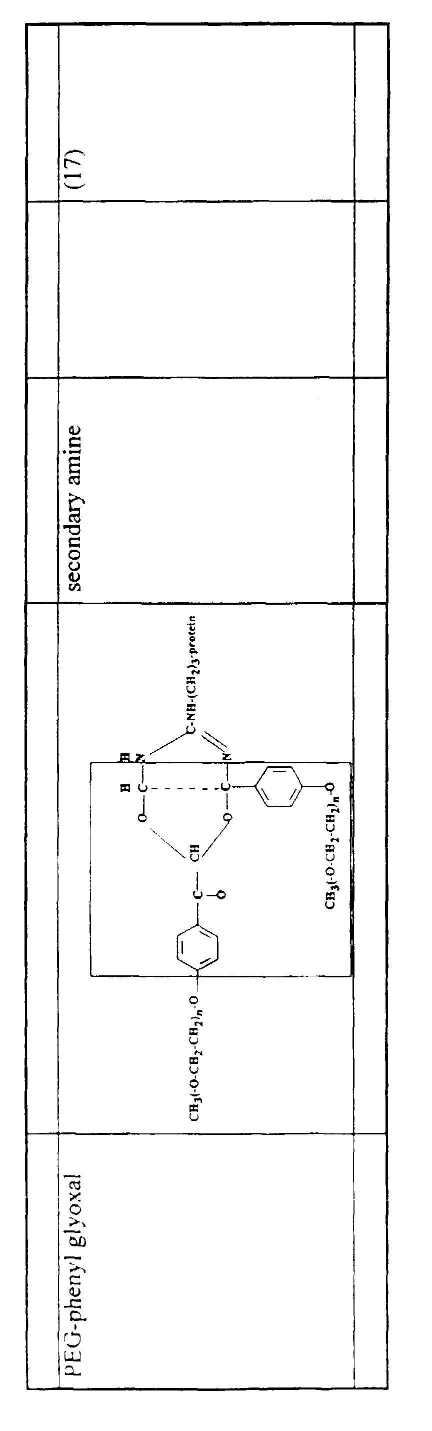

**Table 2**

| ^{**I25**}**I-PEG-CPG2 distribution in LS174T xenografts** |
|---|
| 24 hrs % ID/g tissue |
| 1.61 ± 0.3 |
| 0.63 ± 0.07 |
| 0.98 ± 0.15 |
| 0.58 ± 0.09 |
| 0.36 ± 0.06 |
| 0.14 ± 0.038 |
| 1.81 ± 0.47 |

| 72 hrs |
|---|
| 0.38 ± 0.08 |
| 0.26 ± 0.04 |
| 0.38 ± 0.06 |
| 0.14 ± 0.025 |
| 0.10 ± 0.02 |
| 0.03 ± 0.01 |
| 1.2 ± 0.21 |

### REFERENCES

Abraham, R. *et al* (1994) *Cell Biophysics* **24/25,** 127.

Abuchowski, A. *et al* (1977) "Effect of covalent attachment of polyethylene glycol on immunogenicity and circulating life of bovine liver catalase" *J. Biol. Chem.* **252,** 3582-3586.

Azoulay, M. *et al* (1995) *Anti-cancer Drug Design* **10,** 441-450.

Bagshawe (1987) *Br. J. Cancer* **56,** 531-2; (1989) **60,** 275-281.

Bagshawe, K.D. *et al* (1988) "A cytotoxic agent can be generated selectively at cancer sites" *Br. J. Cancer.* **58,** 700-703.

Blair, A.H. & Ghose, T.I. (1983) "Linkage of cytotoxic agents to immunoglobulin" *J. Immunol. Methods* 59, 129.

Blakey, D.C. *et al* (1996) "2D2767, an improved system for antibody-directed enzyme prodrug therapy that results in tumour regressions in colorectal tumour xenografts" *Cancer Res.* **56,** 3287-3292.

Bosslet, K. *et al* (1994) *Cancer Research* **54,** 2151.

Bradford, M.M. (1976) *Analyt. Biochem.* **72,** 248.

Cobb, L.M. (1989) "Intratumour factors influencing the access of antibody to tumour cells" *Cancer Immunol. Immunother.* **28,** 235-240.

Corvalan *et al* (1987) *Cancer Immunol. Immunother.* **24,** 127-132, 133-137 and 138-143.

Dubois, M. *et al* (1956) *Analyt. Chem.* **28,** 350.

Duncan, R. *et al* (1988) "Anti-cancer agents coupled to N-(2-hydroxypropyl)metha-acrylamide copolymers. 11 Evaluation of daunomycin conjugate in vivo against L1210 leukaemia" *Br. J. Cancer* **57,** 147-156.

Duncan, R. *et al* (1996) "The role of polymer conjugates in the diagnosis and treatment of cancer" in *STP Pharma Sciences* **6,** 237-263.

Duran-Reynolds, F. (1939) "Studies on the localisation of dye and foreign particles in normal and malignant tissues" *Amer. J. Cancer* **35,** 98.

Dykes, P.W. *et al* (1987) "Radioimmunotherapy of cancer: clinical studies and limiting factors" *Cancer Treat. Rev.* **14,** 87-106.

Eno-Amooquaye, E.A. *et al* (1996) "Altered biodistribution of antibody-enzyme conjugate modified with polyethylene glycol" *Br. J. Cancer* **73,** 1323-1327.

Garnett, M.C. & Baldwin, R.W. (1986) "An improved synthesis of a methotrexate-albumin;791T/36 monoclonal antibody conjugate cytotoxic to human osteogenic sarcoma cell lines" *Cancer Res.* **46,** 2407-2412.

Gesson, J.P. *et al* (1994) *Anti-cancer Drug Design* **9**, 409-423.

Hurrell, J.G.R. (CRC Press, 1982) "Monoclonal Hybridoma Antibodies: Techniques and Applications".
Jain, R.K. (1989) "Delivery of novel therapeutic agents in tumours: Physiological barriers and strategies" J. *Nat. Cancer Inst.* **81,** 570-576.

Jungheim, L.N. & Shepherd, T.A. (1994) "Design of anti-tumour prodrugs substrates for antibody targeted enzymes" *Chem. Rev.* **94,** 1553-1566.

Knox, R.J. *et al* (1993) *Cancer and Metastasis Reviews* **12,** 195.

Köhler & Milstein (1975) *Nature* **256,** 495.

Kuefner *et al* (1989) *Biochemistry* **28,** 2288-2297.

Mach *et al* (1980) "Tumour localisation of radiolabelled antibody against carcinoembryonic antigen in patients with carcinoma: A critical evaluation" *New Eng. J. Med.* **303**, 5-10.

Mauger, A.B. *et al* (1994) *J. Med. Chem.* **37,** 3452.

Matsumura. Y. & Macda H. (1986) "A new concept in macromolecule therapeutics in cancer chemotherapy: mechanism of tumortropic accumulation of proteins and the antitumor agents SMANCS" in *Cancer Res.* **6,** 6387-6392.

Melton *et al* (1987) *Biochem. Pharm.* **36**(1), 105-112.

Melton *et al* (1987) *Biochem. Pharm.* **36**(1), 113-121.

Napier, M.P. *et al* (in press) "A phase I tumour site specific study of antibody directed enzyme prodrug therapy (ADEPT) in recurrent or metastatic colorectal carcinoma with pharmacokinetic analysis".

O'Sullivan *et al* (1979) *Anal. Biochem.* **100**, 100-108.

Rodrigues, M.L. *et al* (1995) *Chemistry & Biology* **2**, 223.

Roffler, S.R. *et al* (1991) *Biochem. Pharmacol.* **42**, 2062.

Searle, F. *et al* (1981) "A human choriocarcinoma xenograft in nude mice: a model for the study of antibody localisation" *Br. J. Cancer* **44**, 137-144.

Senter, P.D. *et al* (1988) "Anti-tumor effects of antibody-alkaline phosphatase conjugates in combination with etoposide phosphate" *Proc. Natl. Acad. Sci. USA* **85**, 4842-4846.

Sharma, S.K. *et al* (1992) "Human immune response to monoclonal antibody-enzyme conjugates in ADEPT pilot clinical trial" *Cell Biophysics* **21**, 109-120.

Sharma, S.K. *et al* (1994) "Galactosylated antibodies and antibody enzyme conjugates in antibody directed enzyme prodrug therapy" *Cancer* **73,** 1114-1120.

Szerkerke, M. & Driscoll, J.S. (1977) "The use of macromolecules as carriers of anti-tumour drugs" *Europ. J. Cancer* **13,** 529-537.

Wang, S.M. *et* al (1992) *Cancer Research* **52**, 4484.

Wileman *et al* (1986) "Soluble asparaginase-dextran conjugate show increased circulatory persistence and lowered antigen reactivity" J. *Pharm. Pharmacol.* **38,** 264-271.

Zola, H. (CRC Press, 1988) "Monoclonal Antibodies: A manual of techniques".

### REFERENCES TO TABLE 1

(1) Abuchowski *et al* (1977) *Journal of Biological Chemistry* **252,** 3578-3581.
(2) Pyatak *et al* (1980) *Res. Commun. Chem. Pathol. Pharmacol.* **29,** 113-127.
(3) Yashimoto *et al* (1986) *Jpn. J Can. Res.* **77,** 1264-1270.
(4) Abuchowski *et al* (1984) *Cancer Biochem Biophys.* **7,** 175-186.
(5) Leonard *et al* (1984) *Tetrahedron* **40,** 1585-1590.
(6) Katre *et al* (1987) *Proc. Natl. Acad. Sci. USA* **84,** 1487-1491.
(7) *Wie et al* (1981) *Int. Arch. Allergy. Appl Immunol. 64,* 84-99.
(8) Veronese *et al* (1985) *Appl. Biochem. Biotechnol.* **11**, 141-152.
(9) Beauchamp *et al* (1983) *Analytical Biochemistry* **131**, 25-33.
(10) Pizzo (1991) *Advanced Drug Delivery Reviews* **6,** 153-166.
(11) Zalipsky (1985) WO 90/13540.
(12) Yoshimoto *et al* (1987) *Biochemical and Biophysical Research Communications* **148**, 876-882.
(13) Aldwin & Nitecki (1987) *Analytical Biochemistry* **164,** 494-501.
(14) Goodson & Katre (1990) *Biotechnology* **8,** 343-346.
(15) Harris *et al* (1989) *Applications in Cell Biology and Biotechnology* (pp 203-210) London: Plenum Press.
(16) Delgado *et al* (1990) *Biotechnology and Applied Biochemistry* **12,** 119-128.
(17) Maeda *et al* (1989) EPA 0 340741.

## Claims

1. A three component therapeutic system comprising
(i) a first compound,
(ii) a catalytic macromolecule which does not specifically bind to a tumour antigen but which is capable, following administration to the vascular compartment of a mammal, of being taken up by a tumour in the mammal and is capable of converting the first compound into a second compound, and
(iii) an inhibitor which, following administration to the said vascular compartment, reduces the level of the said catalytic conversion activity in the vascular compartment and normal (non-tumour) tissues.

2. A system according to Claim 1 wherein the catalytic macromolecule is a conjugate or fusion of (i) an enzyme or part thereof and (ii) a macromolecule to the exclusion of transferrin; the conjugate or fusion molecule being at least MW 60 000.

3. A system according to Claim 2 wherein the macromolecule is a polyethylene glycol; a dextran; a polyamino acid; a non-tumour-specific protein, such as an immunoglobulin, an albumin; or hydroxypropyl methylacrylamide.

4. A system according to any one of Claims 1 to 3, wherein the macromolecule comprises sub-units which are not biodegradable linked by biodegradable linking units.

5. A system according to any one of the preceding claims wherein the inhibitor is an enzyme-inactivating small compound.

6. A system according to any one of Claims 1 to 4 wherein the inhibitor is an antibody.

7. A system according to Claim 6 wherein the antibody inhibitor is polygalactosylated.

8. A system according to any one of the preceding claims wherein the catalytic macromolecule is internalised by the tumour cells and is more capable of effecting the said conversion in the presence of an endogenous intracellular co-factor than in its absence.

9. A system according to any one of the preceding claims wherein the first compound is a pro-drug and the second compound is more cytotoxic than the first compound.

10. A system according to any one of Claims 1 to 8 wherein the first compound is a metabolite.

11. A four component system comprising a three component system according to Claim 10 and additionally an antimetabolite cytotoxic compound, the cytotoxicity of which is reduced more by the presence of the said first compound than by the presence of the said second compound.

12. Use of a catalytic macromolecule which does not specifically bind to a tumour antigen but which is capable, following administration to the vascular compartment of a mammal, of being taken up by a tumour in the mammal and is capable of converting a first compound into a second compound in the manufacture of a medicament for combatting a tumour in a patient, wherein the patient has been, is being or will be administered a first compound, and has been, is being or will be administered an inhibitor which, following administration to the said vascular compartment, reduces the level of the said catalytic conversion activity in the vascular compartment and normal (non-tumour) tissues.

13. The use according to Claim 12 wherein the catalytic macromolecule is a conjugate or fusion or (i) an enzyme or part thereof and (ii) a macromolecule to the exclusion of transferrin; the conjugate or fusion molecule being at least MW 60 000.

14. The use according to Claim 13 wherein the macromolecule is a polyethylene glycol; a dextran; a polyamino acid; a non-tumour-specific protein, such as an immunoglobulin, an albumin or hydroxypropyl methylacrylamide.

## Patentansprüche

1. Dreikomponentiges therapeutisches System, enthaltend
(i) eine erste Verbindung,
(ii) ein katalytisches Makromolekül, welches nicht spezifisch an ein Tumorantigen bindet, jedoch nach Eingeben in das Gefäßsystem eines Säugetiers in der Lage ist, durch einen Tumor im Säugetier aufgenommen zu werden und die erste Verbindung in eine zweite Verbindung umzuwandeln, und
(iii) einen Inhibitor, welcher nach Eingeben in das Gefäßsystem das Aktivitätsniveau der katalytischen Umwandlung im Gefäßsystem und im normalen (nicht tumorösen) Gewebe reduziert.

2. System nach Anspruch 1,
wobei das katalytische Makromolekül ein Konjugat oder eine Verschmelzung von (i) einem Enzym oder einem Teil hiervon und (ii) einem Makromolekül mit Ausnahme von Transferrin ist, wobei das Molekül des Konjugats oder der Verschmelzung mindestens MW 60 000 hat.

3. System nach Anspruch 2,
wobei das Makromolekül ein Polyäthylenglykol; ein Dextran; eine Polyaminosäure; ein nicht-tumorspezifisches Protein wie immunglobulin, ein Albumin; oder ein Hydroxypropyl-methylacrylamid ist.

4. System nach mindestens einem der Ansprüche 1 bis 3, wobei das Makromolekül biologisch nicht abbaubare Untereinheiten umfasst, die durch biologisch abbaubare Verbindungseinheiten verbunden sind.

5. System nach mindestens einem der vorangegangenen Ansprüche, wobei der Inhibitor eine Enzym-inaktivierende kleine Verbindung ist.

6. System nach mindestens einem der Ansprüche 1 bis 4, wobei der Inhibitor ein Antikörper ist.

7. System nach Anspruch 6, wobei der Antikörper-Inhibitor polygalaktosyliert ist.

8. System nach mindestens einem der vorhergegangenen Ansprüche, wobei das katalytische Makromolekül von den Tumorzellen aufgenommen wird und wobei es in Anwesenheit eines endogenen intrazellulären Kofaktors eher in der Lage ist, die genannte Umwandlung auszuführen, als in dessen Abwesenheit.

9. System nach mindestens einem der vorangegangenen Ansprüche, wobei die erste Verbindung ein Prodrug und die zweite Verbindung zytotoxischer als die erste Verbindung ist.

10. System nach mindestens einem der Ansprüche 1 bis 8, wobei die erste Verbindung ein Metabolit ist.

11. Vierkomponentensystem enthaltend ein Dreikomponentensystem nach Anspruch 10 und zusätzlich eine antimetabolische zytotoxische Verbindung, deren Zytotoxizität durch die Anwesenheit der ersten Verbindung stärker reduziert wird als durch die Anwesenheit der zweiten Verbindung.

12. Verwendung eines katalytischen Makromoleküls, welches nicht spezifisch an ein Tumorantigen bindet, welches jedoch nach Eingeben in das Gefäßsystems eines Säugetieres in der Lage ist, von einem Tumor im Säugetier aufgenommen zu werden und eine erste Verbindung in eine zweite Verbindung umzuwandeln, zur Herstellung eines Medikaments gegen einen Tumor in einem Patienten, wobei dem Patienten eine erste Verbindung verabreicht worden ist, verabreicht wird oder verabreicht werden wird und ein Inhibitor verabreicht worden ist, verabreicht wird oder verabreicht werden wird, welcher nach Eingeben in das Gefäßsystem des Aktivitätsniveau der katalytischen Umwandlung im Gefäßsystem und im normalen (nicht tumorösen) Gewebe reduziert.

13. Verwendung nach Anspruch 12, wobei das katalytische Makromolekül ein Konjugat oder eine Verschmelzung von (i) einem Enzym oder einem Teil hiervon und (ii) einem Makromolekül mit Ausnahme von Transferrin ist, wobei das Molekül des Konjugats oder der Verschmelzung mindestens MW 60 000 hat.

14. Verwendung nach Anspruch 13, wobei das Makromolekül ein Polyäthylenglykol; ein Dextran; eine Polyaminosäure; ein nicht-tumorspezifisches Protein wie immunglobulin, ein Albumin; oder ein Hydroxypropyl-methylacrylamid ist.

## Revendications

1. Système thérapeutique à trois composants comprenant
(i) un premier composé,
(ii) une macromolécule catalytique qui ne se fixe pas spécifiquement à un antigène tumoral mais qui est capable, après administration au compartiment vasculaire d'un mammifère, d'être absorbée par une tumeur dans le mammifère et est capable de convertir le premier composé en un deuxième composé, et
(iii) un inhibiteur qui, après administration audit compartiment vasculaire, réduit 1c niveau de ladite activité de conversion catalytique dans le compartiment vasculaire et les tissus normaux (non tumoraux).

2. Système selon la revendication 1, dans lequel la macromolécule catalytique est un conjugué ou une fusion dc (i) une enzyme ou une partie de celle-ci et (ii) une macromolécule, à l'exclusion de la transferrine, la molécule conjuguée ou de fusion ayant une masse moléculaire d'au moins 60 000.

3. Système selon la revendication 2, dans lequel la macromolécule est un polyéthylèneglycol ; un dextrane ; un poly(acide aminé) ; une protéine non spécifique d'une tumeur, telle qu'une immunoglobuline, une albumine ; ou l'hydroxypropylméthylacrylamide.

4. Système selon l'une quelconque des revendications 1 à 3, dans lequel la macromolécule comprend des sous-unités qui ne sont pas biodégradables liées par des unités de liaison biodégradables.

5. Système selon l'une quelconque des revendications précédentes, dans lequel l'inhibiteur est un petit composé inactivant les enzymes.

6. Système selon l'une quelconque des revendications 1 à 4, dans lequel l'inhibiteur est un anticorps.

7. Système selon la revendication 6, dans lequel l'inhibiteur anticorps est polygalactosylé.

8. Système selon l'une quelconque des revendications précédentes, dans lequel la macromolécule catalytique est intériorisée par les cellules tumorales et est davantage capable d'effectuer ladite conversion en présence d'un co-facteur intracellulaire endogène qu'en son absence.

9. Système selon l'une quelconque des revendications précédentes, dans lequel le premier composé est un pro-médicament et le deuxième composé est plus cytotoxique que le premier composé.

10. Système selon l'une quelconque des revendications 1 à 8, dans lequel le premier composé est un métabolite.

11. Système à quatre composants comprenant un système à trois composants selon la revendication 10 et de plus un composé cytotoxique anti-métabolite, dont la cytotoxicité est réduite davantage par la présence dudit premier composé que par la présence dudit deuxième composé.

12. Utilisation d'une macromolécule catalytique qui ne se fixe pas spécifiquement à un antigène tumoral mais qui est capable, après administration au compartiment vasculaire d'un mammifère, d'être absorbée par une tumeur dans le mammifère et est capable de convertir un premier composé en un deuxième composé, dans la fabrication d'un médicament pour combattre une tumeur chez un patient, dans laquelle le patient a reçu, reçoit ou recevra un premier composé et a reçu, reçoit ou recevra un inhibiteur qui, après administration audit compartiment vasculaire, réduit le niveau de ladite activité de conversion catalytique dans le compartiment vasculaire et les tissus normaux (non tumoraux).

13. Utilisation selon la revendication 12, dans laquelle la macromolécule catalytique est un conjugué ou une fusion de (i) une enzyme ou une partie de celle-ci et (ii) une macromolécule, à l'exclusion de la transferrine, la molécule conjuguée ou de fusion ayant une masse moléculaire d'au moins 60 000.

14. Utilisation selon la revendication 13, dans laquelle la macromolécule est un polyéthylèneglycol ; un dextrane ; un poly(acide aminé) ; une protéine non spécifique d'une tumeur, telle qu'une immunoglobuline, une albumine ; ou l'hydroxypropylméthylacrylamide.
